# EUROPEAN PATENT APPLICATION

(11) **EP 3 354 664 A1**
(43) Date of publication of application: **01.08.2018**
(21) Application number: 16849046.4
(22) Date of filing: 26.09.2016
(51) Int. Cl.: C07K 19/00

(54) **PROTEIN CONJUGATE COMPRISING MULTIPLE BIOACTIVE POLYPEPTIDES AND IMMUNOGLOBULIN FC REGIONS**

(30) Priority: 25.09.2015 KR 20150136656
(71) Applicant: Hanmi Pharm. Co., Ltd., Hwaseong-si, Gyeonggi-do 18536 (KR)
(72) Inventor: LEE, Jong Soo, Hwaseong-si Gyeonggi-do 18469 (KR); CHOI, Seong Ho, Hwaseong-si Gyeonggi-do 18469 (KR); CHOI, Jae Hyuk, Hwaseong-si Gyeonggi-do 18469 (KR); KIM, Sang Yun, Hwaseong-si Gyeonggi-do 18469 (KR); JUNG, Sung Youb, Hwaseong-si Gyeonggi-do 18469 (KR); KWON, Se Chang, Hwaseong-si Gyeonggi-do 18469 (KR)
(74) Representative: Andrews, Robert
(86) International application number: PCT/KR2016/010745
(87) International publication number: WO 2017/052321

(57) **Abstract**

The present invention relates to a physiologically active polypeptide conjugate comprising multiple physiologically active polypeptides and immunoglobulin Fc regions, use thereof, and a preparation method thereof.

<110>     HANMI PHARM. CO., LTD.

<120>     A protein conjugate comprising multiple physiological
          polypeptides and an immunoglobulin Fc region

<130>     OPA16206-PCT

<150>     KR 10-2015-0136656
<151>     2015-09-25

<160>     69

<170>     KopatentIn 2.0

<210>     1
<211>     29
<212>     PRT
<213>     homo sapiens

<400>     1
His Ser Gln Gly Thr Phe Thr Ser Asp Tyr Ser Lys Tyr Leu Asp Ser
  1               5                  10                  15

Arg Arg Ala Gln Asp Phe Val Gln Trp Leu Met Asn Thr
             20                  25



<210>     2
<211>     29
<212>     PRT
<213>     Artificial Sequence

<220>
<223>     glucagon analog


<400>     2
His Ser Gln Gly Thr Phe Thr Ser Asp Tyr Ser Lys Tyr Leu Asp Cys
  1               5                  10                  15

Asp Arg Ala Gln Asp Phe Val Gln Trp Leu Met Asn Thr
             20                  25



<210>     3
<211>     29
<212>     PRT
<213>     Artificial Sequence

<220>
<223>     glucagon analog


<400>     3
His Ser Gln Gly Thr Phe Thr Ser Asp Tyr Ser Lys Tyr Leu Asp Cys
  1               5                  10                  15

Glu Arg Ala Gln Asp Phe Val Gln Trp Leu Met Asn Thr
             20                  25



<210>     4
<211>     29

<212>    PRT
<213>    Artificial Sequence

<220>
<223>    glucagon analog


<400>    4
His Ser Gln Gly Thr Phe Thr Ser Asp Tyr Ser Lys Tyr Leu Asp Ser
1               5                   10                  15

Cys Asp Ala Gln Asp Phe Val Gln Trp Leu Met Asn Thr
            20                  25


<210>    5
<211>    29
<212>    PRT
<213>    Artificial Sequence

<220>
<223>    glucagon analog


<400>    5
His Ser Gln Gly Thr Phe Thr Ser Asp Tyr Ser Lys Tyr Leu Asp Ser
1               5                   10                  15

Cys Glu Ala Gln Asp Phe Val Gln Trp Leu Met Asn Thr
            20                  25


<210>    6
<211>    29
<212>    PRT
<213>    Artificial Sequence

<220>
<223>    glucagon analog


<400>    6
His Ser Gln Gly Thr Phe Thr Ser Asp Tyr Ser Lys Tyr Leu Asp Ser
1               5                   10                  15

Cys Glu Ala Asp Asp Phe Val Gln Trp Leu Met Asn Thr
            20                  25


<210>    7
<211>    29
<212>    PRT
<213>    Artificial Sequence

<220>
<223>    glucagon analog


<400>    7
Tyr Ser Gln Gly Thr Phe Thr Ser Asp Tyr Ser Lys Tyr Leu Asp Ser
1               5                   10                  15

Cys Glu Ala Asp Asp Phe Val Gln Trp Leu Met Asn Thr

20                  25


<210>   8
<211>   29
<212>   PRT
<213>   Artificial Sequence

<220>
<223>   glucagon analog


<220>
<221>   MISC_FEATURE
<222>   (2)
<223>   Xaa is aminoisobutyric acid (Aib)


<400>   8
Tyr Xaa Gln Gly Thr Phe Thr Ser Asp Tyr Ser Lys Tyr Leu Asp Ser
1               5                   10                  15

Cys Asp Ala Gln Asp Phe Val Gln Trp Leu Ile Asn Thr
            20                  25


<210>   9
<211>   29
<212>   PRT
<213>   Artificial Sequence

<220>
<223>   glucagon analog


<220>
<221>   MISC_FEATURE
<222>   (2)
<223>   Xaa is aminoisobutyric acid (Aib)


<400>   9
Tyr Xaa Gln Gly Thr Phe Thr Ser Asp Tyr Ser Lys Tyr Leu Asp Ser
1               5                   10                  15

Cys Asp Ala Gln Asp Phe Val Val Trp Leu Ile Asn Thr
            20                  25


<210>   10
<211>   29
<212>   PRT
<213>   Artificial Sequence

<220>
<223>   glucagon analog


<220>
<221>   MISC_FEATURE
<222>   (2)
<223>   Xaa is aminoisobutyric acid (Aib)

<400>    10
Tyr Xaa Gln Gly Thr Phe Thr Ser Asp Tyr Ser Lys Tyr Leu Asp Ser
1               5                   10                  15

Cys Asp Ala Asp Asp Phe Val Val Trp Leu Ile Asn Thr
            20                  25

<210>    11
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<400>    11
Tyr Xaa Gln Gly Thr Phe Thr Ser Asp Tyr Ser Lys Tyr Leu Asp Glu
1               5                   10                  15

Lys Cys Ala Lys Glu Phe Val Gln Trp Leu Met Asn Thr
            20                  25

<210>    12
<211>    30
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    12
Tyr Xaa Gln Gly Thr Phe Thr Ser Asp Tyr Ser Lys Tyr Leu Asp Glu
1               5                   10                  15

Lys Arg Ala Lys Glu Phe Val Gln Trp Leu Met Asn Thr Cys
            20                  25                  30

<210>    13
<211>    29

<212>    PRT
<213>    Artificial Sequence

<220>
<223>    glucagon analog


<220>
<221>    MISC_FEATURE
<222>    (2)
<223>    Xaa is aminoisobutyric acid (Aib)


<400>    13
Tyr Xaa Gln Gly Thr Phe Thr Ser Asp Tyr Ser Cys Tyr Leu Asp Ser
1               5                   10                  15

Arg Arg Ala Gln Asp Phe Val Gln Trp Leu Met Asn Thr
            20                  25


<210>    14
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<400>    14
Tyr Xaa Gln Gly Thr Phe Thr Ser Asp Tyr Ser Lys Tyr Leu Asp Cys
1               5                   10                  15

Lys Arg Ala Lys Glu Phe Val Gln Trp Leu Met Asn Thr
            20                  25


<210>    15
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<400>    15
Tyr Xaa Gln Gly Thr Phe Thr Ser Asp Tyr Ser Lys Tyr Leu Cys Glu
1               5                   10                  15

Lys Arg Ala Gln Asp Phe Val Val Trp Leu Met Asn Thr
            20                  25

<210>    16
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<400>    16
Tyr Xaa Gln Gly Thr Phe Thr Ser Asp Tyr Ser Lys Tyr Leu Asp Cys
 1               5                  10                  15

Arg Arg Ala Gln Val Phe Val Gln Trp Leu Met Arg Thr
            20                  25

<210>    17
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<400>    17
Tyr Xaa Gln Gly Thr Phe Thr Ser Asp Tyr Ser Lys Tyr Leu Asp Cys
 1               5                  10                  15

Val Arg Ala Gln Asp Phe Val Gln Trp Leu Met Arg Thr
            20                  25

<210>    18
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<400>    18
Tyr Xaa Gln Gly Thr Phe Thr Ser Asp Tyr Ser Lys Tyr Leu Asp Ser
1               5                   10                  15

Arg Arg Ala Cys Asp Phe Arg Leu Trp Leu Met Asn Thr
            20                  25

<210>    19
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    19
Tyr Xaa Gln Gly Thr Phe Thr Ser Asp Tyr Ser Lys Tyr Leu Cys Glu
1               5                   10                  15

Lys Arg Ala Lys Glu Phe Val Gln Trp Leu Met Asn Thr
            20                  25

<210>    20
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    20
Tyr Xaa Gln Gly Thr Phe Thr Ser Asp Tyr Ser Lys Tyr Leu Asp Glu
1               5                   10                  15

Cys Arg Ala Lys Glu Phe Val Gln Trp Leu Met Asn Thr
            20                  25

<210>    21
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    21
Tyr Xaa Gln Gly Thr Phe Thr Ser Asp Tyr Ser Lys Tyr Leu Asp Glu
 1               5                  10                  15

Lys Cys Ala Lys Glu Phe Val Gln Trp Leu Met Asn Thr
            20                  25

<210>    22
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    22
Tyr Xaa Gln Gly Thr Phe Thr Ser Asp Tyr Ser Lys Tyr Leu Asp Glu
 1               5                  10                  15

Lys Arg Cys Lys Glu Phe Val Gln Trp Leu Met Asn Thr
            20                  25

<210>    23
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    23
Tyr Xaa Gln Gly Thr Phe Thr Ser Asp Tyr Ser Lys Tyr Cys Asp Glu
1               5                   10                  15

Lys Arg Ala Lys Glu Phe Val Gln Trp Leu Met Asn Thr
            20                  25


<210>    24
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    24
Tyr Xaa Gln Gly Thr Phe Thr Ser Asp Tyr Ser Lys Cys Leu Asp Glu
1               5                   10                  15

Lys Arg Ala Lys Glu Phe Val Gln Trp Leu Met Asn Thr
            20                  25


<210>    25
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    25
Tyr Xaa Gln Gly Thr Phe Thr Ser Asp Tyr Ser Lys Tyr Leu Asp Glu
1               5                   10                  15

Lys Arg Ala Lys Cys Phe Val Gln Trp Leu Met Asn Thr
            20                  25


<210>    26
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    26
Trp Xaa Gln Gly Thr Phe Thr Ser Asp Tyr Ser Lys Tyr Leu Asp Glu
1               5                   10                  15

Cys Arg Ala Lys Asp Phe Val Gln Trp Leu Met Asn Thr
            20                  25


<210>    27
<211>    29
<212>    PRT
<213>    Artificial Sequence

<220>
<223>    glucagon analog


<220>

<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    27
Tyr Xaa Gln Gly Thr Phe Val Ser Asp Tyr Ser Lys Tyr Leu Asp Glu
1               5                   10                  15

Cys Arg Ala Lys Asp Phe Val Gln Trp Leu Met Asn Thr
            20                  25


<210>    28
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    28
Trp Xaa Gln Gly Thr Phe Val Ser Asp Tyr Ser Lys Tyr Leu Asp Glu
1               5                   10                  15

Cys Arg Ala Lys Asp Phe Val Gln Trp Leu Met Asn Thr
            20                  25


<210>    29
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    29
Tyr Xaa Gln Gly Thr Phe Thr Ser Asp Tyr Ser Lys Cys Leu Asp Glu
1               5                   10                  15

Arg Arg Ala Lys Asp Phe Val Gln Trp Leu Met Asn Thr
            20                  25

<210>    30
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    30
Trp Xaa Gln Gly Thr Phe Thr Ser Asp Tyr Ser Lys Cys Leu Asp Glu
1               5                   10                  15

Arg Arg Ala Lys Asp Phe Val Gln Trp Leu Met Asn Thr
            20                  25

<210>    31
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (17)..(21)
<223>    amino acids at positions 17 and 21 form a ring

<400>    31
Tyr Xaa Gln Gly Thr Phe Thr Ser Asp Tyr Ser Lys Tyr Leu Asp Cys
1               5                   10                  15

Lys Arg Ala Lys Glu Phe Val Gln Trp Leu Met Asn Thr
            20                  25

<210>    32
<211>    28
<212>    PRT
<213>    Artificial Sequence

<220>
<223>    glucagon analog

<220>
<221>    MISC_FEATURE
<222>    (15)..(19)
<223>    amino acids at positions 15 and 19 form a ring

<400>    32
Ser Gln Gly Thr Phe Thr Ser Asp Tyr Ser Lys Tyr Leu Asp Glu Cys
1               5                   10                  15

Arg Ala Lys Glu Phe Val Gln Trp Leu Met Asn Thr
            20                  25

<210>    33
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<400>    33
Tyr Xaa Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr
            20                  25

<210>    34
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    34
Trp Xaa Gln Gly Thr Phe Thr Ser Asp Tyr Ser Lys Tyr Cys Asp Glu
1               5                   10                  15

Arg Arg Ala Lys Glu Phe Val Gln Trp Leu Met Asn Thr
            20                  25


<210>    35
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    35
Tyr Xaa Gln Gly Thr Phe Thr Ser Asp Tyr Ser Lys Tyr Cys Asp Glu
1               5                   10                  15

Arg Arg Ala Lys Glu Phe Val Gln Trp Leu Met Asn Thr
            20                  25


<210>    36
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring


<400>    36
Tyr Xaa Gln Gly Thr Phe Thr Ser Asp Cys Ser Lys Tyr Leu Asp Glu
1               5                   10                  15

Arg Arg Ala Lys Glu Phe Val Gln Trp Leu Met Asn Thr
            20                  25


<210>    37
<211>    30
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)
<223>    amino acids at positions 16 and 20 form a ring


<400>    37
Tyr Xaa Gln Gly Thr Phe Thr Ser Asp Tyr Ser Lys Tyr Leu Asp Glu
1               5                   10                  15

Arg Arg Ala Lys Glu Phe Val Gln Trp Leu Met Asn Thr Cys
            20                  25                  30


<210>    38
<211>    29
<212>    PRT
<213>    Artificial Sequence

<220>
<223>    glucagon analog


<220>
<221>    MISC_FEATURE
<222>    (2)
<223>    Xaa is aminoisobutyric acid (Aib)


<220>
<221>    MISC_FEATURE
<222>    (16)..(20)

<223>    amino acids at positions 16 and 20 form a ring

<400>    38
Tyr Xaa Gln Gly Thr Phe Cys Ser Asp Tyr Ser Lys Tyr Leu Asp Glu
1               5                   10                  15

Arg Arg Ala Lys Glu Phe Val Gln Trp Leu Met Asn Thr
            20                  25

<210>    39
<211>    29
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    39
Tyr Xaa Gln Gly Thr Phe Val Ser Asp Cys Ser Lys Tyr Leu Asp Glu
1               5                   10                  15

Arg Arg Ala Lys Asp Phe Val Gln Trp Leu Met Asn Thr
            20                  25

<210>    40
<211>    30
<212>    PRT
<213>    Artificial Sequence

<220>
<223>    glucagon analog

<220>
<221>    MISC_FEATURE
<222>    (2)
<223>    Xaa is aminoisobutyric acid (Aib)

<220>
<221>    MISC_FEATURE
<222>    (16)..(20)
<223>    amino acids at positions 16 and 20 form a ring

<400>    40
Tyr Xaa Gln Gly Thr Phe Val Ser Asp Tyr Ser Lys Tyr Leu Asp Glu

1               5                   10                  15

Arg Arg Ala Lys Asp Phe Val Gln Trp Leu Met Asn Thr Cys
            20                  25                  30

<210>   41
<211>   29
<212>   PRT
<213>   Artificial Sequence

<220>
<223>   glucagon analog

<220>
<221>   MISC_FEATURE
<222>   (2)
<223>   Xaa is aminoisobutyric acid (Aib)

<220>
<221>   MISC_FEATURE
<222>   (16)..(20)
<223>   amino acids at positions 16 and 20 form a ring

<400>   41
Tyr Xaa Gln Gly Thr Phe Cys Ser Asp Tyr Ser Lys Tyr Leu Asp Glu
1               5                   10                  15

Arg Arg Ala Lys Asp Phe Val Gln Trp Leu Met Asn Thr
            20                  25

<210>   42
<211>   29
<212>   PRT
<213>   Artificial Sequence

<220>
<223>   glucagon analog

<220>
<221>   MISC_FEATURE
<222>   (2)
<223>   Xaa is aminoisobutyric acid (Aib)

<400>   42
Tyr Xaa Gln Gly Thr Phe Cys Ser Asp Tyr Ser Lys Tyr Leu Asp Ser
1               5                   10                  15

Arg Arg Ala Gln Asp Phe Val Gln Trp Leu Met Asn Thr
            20                  25

<210>   43
<211>   29
<212>   PRT
<213>   Artificial Sequence

<220>
<223>    glucagon analog

<220>
<221>    MISC_FEATURE
<222>    (2)
<223>    Xaa is aminoisobutyric acid (Aib)

<400>    43
Tyr Xaa Gln Gly Thr Phe Thr Ser Asp Cys Ser Lys Tyr Leu Asp Ser
1               5                   10                  15

Arg Arg Ala Gln Asp Phe Val Gln Trp Leu Met Asn Thr
            20                  25

<210>    44
<211>    30
<212>    PRT
<213>    Artificial Sequence

<220>
<223>    glucagon analog

<220>
<221>    MISC_FEATURE
<222>    (2)
<223>    Xaa is aminoisobutyric acid (Aib)

<400>    44
Tyr Xaa Gln Gly Thr Phe Thr Ser Asp Tyr Ser Lys Tyr Leu Asp Ser
1               5                   10                  15

Arg Arg Ala Gln Asp Phe Val Gln Trp Leu Met Asn Thr Cys
            20                  25                  30

<210>    45
<211>    30
<212>    PRT
<213>    Artificial Sequence

<220>
<223>    glucagon analog

<220>
<221>    MISC_FEATURE
<222>    (1)
<223>    Xaa is histidine, desamino-histidyl, N-dimethyl-histidyl,
         beta-hydroxy imidazopropionyl, 4-imidazoacetyl, beta-carboxy
         imidazopropionyl, tryptophan, or tyrosine, or is absent

<220>
<221>    MISC_FEATURE
<222>    (2)
<223>    Xaa is alpha-methyl-glutamic acid, aminoisobutyric acid (Aib),
         D-alanine, glycine, Sar (N-methylglycine), serine, or D-serine

<220>
<221>    MISC_FEATURE
<222>    (7)
<223>    Xaa is threonine, valine, or cysteine


<220>
<221>    MISC_FEATURE
<222>    (10)
<223>    Xaa is tyrosine or cysteine


<220>
<221>    MISC_FEATURE
<222>    (12)
<223>    Xaa is lysine or cysteine


<220>
<221>    MISC_FEATURE
<222>    (13)
<223>    Xaa is tyrosine or cysteine


<220>
<221>    MISC_FEATURE
<222>    (14)
<223>    Xaa is leucine or cysteine


<220>
<221>    MISC_FEATURE
<222>    (15)
<223>    Xaa is aspartic acid, glutamic acid, or cysteine


<220>
<221>    MISC_FEATURE
<222>    (16)
<223>    Xaa is glutamic acid, aspartic acid, serine,
         alpha-methyl-glutamic acid, or cysteine, or is absent;


<220>
<221>    MISC_FEATURE
<222>    (17)
<223>    Xaa is aspartic acid, glutamine, glutamic acid, lysine, arginine,
         serine, cysteine, or valine, or is absent


<220>
<221>    MISC_FEATURE
<222>    (18)
<223>    Xaa is alanine, aspartic acid, glutamic acid, arginine, valine,
         or cysteine, or is absent


<220>
<221>    MISC_FEATURE
<222>    (19)
<223>    Xaa is alanine, arginine, serine, valine, or cysteine, or is

absent

<220>
<221>    MISC_FEATURE
<222>    (20)
<223>    Xaa is lysine, histidine, glutamine, aspartic acid, lysine,
         arginine, alpha-methyl-glutamic acid, or cysteine, or is absent

<220>
<221>    MISC_FEATURE
<222>    (21)
<223>    Xaa is aspartic acid, glutamic acid, leucine, valine, or
         cysteine, or is absent

<220>
<221>    MISC_FEATURE
<222>    (23)
<223>    Xaa is isoleucine, valine, or arginine, or is absent

<220>
<221>    MISC_FEATURE
<222>    (24)
<223>    Xaa is valine, arginine, alanine, cysteine, glutamic acid,
         lysine, glutamine, alpha-methyl-glutamic acid, or leucine, or is
         absent;

<220>
<221>    MISC_FEATURE
<222>    (27)
<223>    Xaa is isoleucine, valine, alanine, lysine, methionine,
         glutamine, or arginine, or is absent

<220>
<221>    MISC_FEATURE
<222>    (28)
<223>    Xaa is glutamine, lysine, asparagine, or arginine, or is absent

<220>
<221>    MISC_FEATURE
<222>    (29)
<223>    Xaa is lysine, alanine, glycine, or threonine, or is absent

<220>
<221>    MISC_FEATURE
<222>    (30)
<223>    Xaa is cysteine, or is absent

<400>    45
Xaa Xaa Gln Gly Thr Phe Xaa Ser Asp Xaa Ser Xaa Xaa Xaa Xaa Xaa
1               5                   10                  15

Xaa Xaa Xaa Xaa Xaa Phe Xaa Xaa Trp Leu Xaa Xaa Xaa Xaa
            20                  25                  30

<210>    46
<211>    180
<212>    PRT
<213>    Artificial Sequence

<220>
<223>    FGF21 dHis/M168A analog


<400>    46
Pro Ile Pro Asp Ser Ser Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ser Tyr Ala Ser
            180


<210>    47
<211>    179
<212>    PRT
<213>    Artificial Sequence

<220>
<223>    FGF21 dHis/dPro/S167T analog


<400>    47
Ile Pro Asp Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
            35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Thr Met Val Gly Pro Ser Gln Gly Arg Ser Pro Ser
                165                 170                 175

Tyr Ala Ser

<210>   48
<211>   179
<212>   PRT
<213>   Artificial Sequence

<220>
<223>   FGF21 dHis/dPro/M168A analog

<400>   48
Ile Pro Asp Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
            35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ser
                165                 170                 175

Tyr Ala Ser

<210>    49
<211>    179
<212>    PRT
<213>    Artificial Sequence

<220>
<223>    FGF21 dHis/dPro/M168F analog

<400>    49
Ile Pro Asp Ser Ser Pro Leu Leu Gln Phe Gly Gly Gln Val Arg Gln
  1               5                  10                  15

Arg Tyr Leu Tyr Thr Asp Asp Ala Gln Gln Thr Glu Ala His Leu Glu
             20                  25                  30

Ile Arg Glu Asp Gly Thr Val Gly Gly Ala Ala Asp Gln Ser Pro Glu
         35                  40                  45

Ser Leu Leu Gln Leu Lys Ala Leu Lys Pro Gly Val Ile Gln Ile Leu
     50                  55                  60

Gly Val Lys Thr Ser Arg Phe Leu Cys Gln Arg Pro Asp Gly Ala Leu
 65                  70                  75                  80

Tyr Gly Ser Leu His Phe Asp Pro Glu Ala Cys Ser Phe Arg Glu Leu
                 85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Phe Val Gly Pro Ser Gln Gly Arg Ser Pro Ser
                165                 170                 175

Tyr Ala Ser

<210>    50
<211>    180
<212>    PRT
<213>    Artificial Sequence

<220>
<223>    FGF21 dHis/D5A/M168A analog


<400>    50
Pro Ile Pro Ala Ser Ser Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ser Tyr Ala Ser
            180


<210>    51
<211>    180
<212>    PRT
<213>    Artificial Sequence

<220>
<223>    FGF21 dHis/S6A/M168A analog


<400>    51
Pro Ile Pro Asp Ala Ser Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ser Tyr Ala Ser
            180


<210>   52
<211>   180
<212>   PRT
<213>   Artificial Sequence

<220>
<223>   FGF21 dHis/S7A/M168A analog


<400>   52
Pro Ile Pro Asp Ser Ala Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala

115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ser Tyr Ala Ser
            180


<210>    53
<211>    180
<212>    PRT
<213>    Artificial Sequence

<220>
<223>    FGF21 dHis/P8A/M168A analog


<400>    53
Pro Ile Pro Asp Ser Ser Ala Leu Leu Gln Phe Gly Gly Gln Val Arg
 1               5                  10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ser Tyr Ala Ser
            180


<210>    54

<211>    180
<212>    PRT
<213>    Artificial Sequence

<220>
<223>    FGF21 dHis/L9A/M168A analog


<400>    54
Pro Ile Pro Asp Ser Ser Pro Ala Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ser Tyr Ala Ser
            180


<210>    55
<211>    180
<212>    PRT
<213>    Artificial Sequence

<220>
<223>    FGF21 dHis/S172A/M168A analog


<400>    55
Pro Ile Pro Asp Ser Ser Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro

35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ala Gln Gly Arg Ser Pro
                165                 170                 175

Ser Tyr Ala Ser
            180


<210>    56
<211>    180
<212>    PRT
<213>    Artificial Sequence

<220>
<223>    FGF21 dHis/S176A/M168A analog


<400>    56
Pro Ile Pro Asp Ser Ser Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ala Pro
                165                 170                 175

Ser Tyr Ala Ser
            180

<210>    57
<211>    179
<212>    PRT
<213>    Artificial Sequence

<220>
<223>    FGF21 dHis/P177A/M168A analog

<400>    57
Pro Ile Pro Asp Ser Ser Pro Leu Leu Gln Phe Gly Gly Gln Val Arg
  1               5                  10                  15

Gln Arg Tyr Leu Tyr Thr Asp Asp Ala Gln Gln Thr Glu Ala His Leu
             20                  25                  30

Glu Ile Arg Glu Asp Gly Thr Val Gly Gly Ala Ala Asp Gln Ser Pro
         35                  40                  45

Glu Ser Leu Leu Gln Leu Lys Ala Leu Lys Pro Gly Val Ile Gln Ile
     50                  55                  60

Leu Gly Val Lys Thr Ser Arg Phe Leu Cys Gln Arg Pro Asp Gly Ala
 65                  70                  75                  80

Leu Tyr Gly Ser Leu His Phe Asp Pro Glu Ala Cys Ser Phe Arg Glu
                 85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Ala
                165                 170                 175

Ser Tyr Ala

<210>    58
<211>    180

<212>    PRT
<213>    Artificial Sequence

<220>
<223>    FGF21 dHis/S178A/M168A analog


<400>    58
Pro Ile Pro Asp Ser Ser Pro Leu Leu Gln Phe Gly Gly Gln Val Arg
1               5                   10                  15

Gln Arg Tyr Leu Tyr Thr Asp Asp Ala Gln Gln Thr Glu Ala His Leu
            20                  25                  30

Glu Ile Arg Glu Asp Gly Thr Val Gly Gly Ala Ala Asp Gln Ser Pro
        35                  40                  45

Glu Ser Leu Leu Gln Leu Lys Ala Leu Lys Pro Gly Val Ile Gln Ile
    50                  55                  60

Leu Gly Val Lys Thr Ser Arg Phe Leu Cys Gln Arg Pro Asp Gly Ala
65                  70                  75                  80

Leu Tyr Gly Ser Leu His Phe Asp Pro Glu Ala Cys Ser Phe Arg Glu
                85                  90                  95

Leu Leu Leu Glu Asp Gly Tyr Asn Val Tyr Gln Ser Glu Ala His Gly
            100                 105                 110

Leu Pro Leu His Leu Pro Gly Asn Lys Ser Pro His Arg Asp Pro Ala
        115                 120                 125

Pro Arg Gly Pro Ala Arg Phe Leu Pro Leu Pro Gly Leu Pro Pro Ala
    130                 135                 140

Leu Pro Glu Pro Pro Gly Ile Leu Ala Pro Gln Pro Pro Asp Val Gly
145                 150                 155                 160

Ser Ser Asp Pro Leu Ser Ala Val Gly Pro Ser Gln Gly Arg Ser Pro
                165                 170                 175

Ala Tyr Ala Ser
            180


<210>    59
<211>    179
<212>    PRT
<213>    Artificial Sequence

<220>
<223>    FGF21 dHis/dPro/D5A/M168A analog


<400>    59
Ile Pro Ala Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ser
                165                 170                 175

Tyr Ala Ser

<210>   60
<211>   179
<212>   PRT
<213>   Artificial Sequence

<220>
<223>   FGF21 dHis/dPro/S6A/M168A analog

<400>   60
Ile Pro Asp Ala Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ser
                165                 170                 175

Tyr Ala Ser

<210>    61
<211>    179
<212>    PRT
<213>    Artificial Sequence

<220>
<223>    FGF21 dHis/dPro/S7A/M168A analog

<400>    61
Ile Pro Asp Ser Ala Pro Leu Leu Gln Phe Gly Gly Gln Val Arg Gln
 1               5                  10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ser
                165                 170                 175

Tyr Ala Ser

<210>    62
<211>    179
<212>    PRT

<213>    Artificial Sequence

<220>
<223>    FGF21 dHis/dPro/P8A/M168A analog


<400>    62
Ile Pro Asp Ser Ser Ala Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ser
                165                 170                 175

Tyr Ala Ser



<210>    63
<211>    179
<212>    PRT
<213>    Artificial Sequence

<220>
<223>    FGF21 dHis/dPro/L9A/M168A analog


<400>    63
Ile Pro Asp Ser Ser Pro Ala Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ser
                165                 170                 175

Tyr Ala Ser

<210>    64
<211>    179
<212>    PRT
<213>    Artificial Sequence

<220>
<223>    FGF21 dHis/dPro/S172A/M168A analog

<400>    64
Ile Pro Asp Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu

130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ala Gln Gly Arg Ser Pro Ser
                165                 170                 175

Tyr Ala Ser

<210>   65
<211>   179
<212>   PRT
<213>   Artificial Sequence

<220>
<223>   FGF21 dHis/dPro/S176A/M168A analog

<400>   65
Ile Pro Asp Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ala Pro Ser
                165                 170                 175

Tyr Ala Ser

<210>   66
<211>   179
<212>   PRT
<213>   Artificial Sequence

<220>
<223>    FGF21 dHis/dPro/P177A/M168A analog


<400>    66
Ile Pro Asp Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu
    50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Ala Ser
                165                 170                 175

Tyr Ala Ser



<210>    67
<211>    179
<212>    PRT
<213>    Artificial Sequence

<220>
<223>    FGF21 dHis/dPro/S178A/M168A analog


<400>    67
Ile Pro Asp Ser Ser Pro Leu Leu Gln Phe Gly Gly Gln Val Arg Gln
1               5                   10                  15

Arg Tyr Leu Tyr Thr Asp Asp Ala Gln Gln Thr Glu Ala His Leu Glu
            20                  25                  30

Ile Arg Glu Asp Gly Thr Val Gly Gly Ala Ala Asp Gln Ser Pro Glu
        35                  40                  45

Ser Leu Leu Gln Leu Lys Ala Leu Lys Pro Gly Val Ile Gln Ile Leu

50                  55                  60

Gly Val Lys Thr Ser Arg Phe Leu Cys Gln Arg Pro Asp Gly Ala Leu
65                  70                  75                  80

Tyr Gly Ser Leu His Phe Asp Pro Glu Ala Cys Ser Phe Arg Glu Leu
                85                  90                  95

Leu Leu Glu Asp Gly Tyr Asn Val Tyr Gln Ser Glu Ala His Gly Leu
            100                 105                 110

Pro Leu His Leu Pro Gly Asn Lys Ser Pro His Arg Asp Pro Ala Pro
        115                 120                 125

Arg Gly Pro Ala Arg Phe Leu Pro Leu Pro Gly Leu Pro Pro Ala Leu
    130                 135                 140

Pro Glu Pro Pro Gly Ile Leu Ala Pro Gln Pro Pro Asp Val Gly Ser
145                 150                 155                 160

Ser Asp Pro Leu Ser Ala Val Gly Pro Ser Gln Gly Arg Ser Pro Ala
                165                 170                 175

Tyr Ala Ser


<210>    68
<211>    21
<212>    PRT
<213>    Artificial Sequence

<220>
<223>    Insulin A-chain


<220>
<221>    MISC_FEATURE
<222>    (1)
<223>    Xaa is alanine, glycine, glutamine, histidine, glutamic acid, or
         asparagine


<220>
<221>    MISC_FEATURE
<222>    (5)
<223>    Xaa is alanine, glutamic acid, glutamine, histidine, or
         asparagine


<220>
<221>    MISC_FEATURE
<222>    (12)
<223>    Xaa is alanine, serine, glutamine, glutamic acid, histidine, or
         asparagine


<220>
<221>    MISC_FEATURE
<222>    (14)
<223>    Xaa is alanine, tyrosine, glutamic acid, histidine, lysine,
         aspartic acid, or asparagine

<220>
<221>    MISC_FEATURE
<222>    (16)
<223>    Xaa is alanine, leucine, tyrosine, histidine, glutamic acid, or
         asparagine

<220>
<221>    MISC_FEATURE
<222>    (19)
<223>    Xaa is alanine, tyrosine, serine, glutamic acid, histidine, or
         asparagine

<220>
<221>    MISC_FEATURE
<222>    (21)
<223>    Xaa is asparagine, glycine, histidine, or alanine

<400>    68
Xaa Ile Val Glu Xaa Cys Cys Thr Ser Ile Cys Xaa Leu Xaa Gln Xaa
1               5                   10                  15

Glu Asn Xaa Cys Xaa
            20

<210>    69
<211>    30
<212>    PRT
<213>    Artificial Sequence

<220>
<223>    Insulin B-chain

<220>
<221>    MISC_FEATURE
<222>    (16)
<223>    Xaa is tyrosine, glutamic acid, or aspartic acid, or is absent

<220>
<221>    MISC_FEATURE
<222>    (25)
<223>    Xaa is phenylalanine, or is absent

<220>
<221>    MISC_FEATURE
<222>    (27)
<223>    Xaa is threonine, or is absent

<220>
<221>    MISC_FEATURE
<222>    (28)
<223>    Xaa is proline, glutamic acid, or aspartic acid, or is absent

<400>    69
Phe Val Asn Gln His Leu Cys Gly Ser His Leu Val Glu Ala Leu Xaa

1                   5                   10                  15
Leu Val Cys Gly Glu Arg Gly Phe Xaa Tyr Xaa Xaa Lys Thr
                20                  25                  30

## Description

### [Technical Field]

The present invention relates to a protein conjugate including multiple physiologically active polypeptides and an immunoglobulin Fc region, a use thereof, and a preparation method thereof.

### [Background Art]

Recent economic advances and lifestyle changes have been accompanied by great changes in dietary habits. Particularly, busy people of today are exposed to high-calorie diets, little exercise, excess stress, irregular lifestyles, etc., which cause an increase in various kinds of adult diseases.

Overweight and obesity are responsible for increasing blood pressure and cholesterol levels and causing or worsening various diseases, such as cardiac diseases, diabetes, arthritis, etc. Nonalcoholic fatty liver disease may lead to liver fibrosis, liver cirrhosis, and liver cancer if risk factors such as overweight, obesity, hyperlipidemia, insulin resistance, diabetes, etc., are not properly handled.

Patients who have at least one adult disease that cause problems in metabolic diseases such as diabetes, obesity, hyperlipidemia, fatty liver, etc., have a high risk that other adult disease(s) may occur, and these patients require simultaneous treatment and control of several adult diseases.

Insulin is a peptide secreted by the pancreatic beta cells and has a very important role in controlling blood glucose levels in the body. A metabolic disease associated with the insufficient amount of insulin secretion or abnormal function, which results in an increase of blood glucose levels, is called diabetes. Defective insulin secretion or an abnormal function of the secreted insulin prevents proper control of *in-vivo* blood glucose levels and causes an increase therein, and this type of diabetes is called type 2 diabetes, whereas when the blood glucose levels increase due to the failure in insulin secretion in the pancreas, this is called type 1 diabetes. Type 2 diabetes is treated using an oral hypoglycemic agent which contains chemical substances as active ingredients, and in some patients, insulin may be used for treatment. In contrast, for the treatment of type 1 diabetes, insulin administration is essential.

Currently, insulin is available as an injection drug. In principle, insulin must be administered by subcutaneous injection and the administration method varies according to the action time. The insulin administration by injection shows a more rapid hypoglycemic effect than the drug for oral administration. The insulin injection can be safely administered even in environments where the drug cannot be used for oral administration, and it does not have any limitation on its dose. However, such insulin therapy requires a continuous use of three-times-daily injections and thus has drawbacks such as rejection of the injection needle by patients, difficulty in administration, symptoms of hypoglycemia, body weight increase due to long-term insulin administration, etc. Additionally, the phenomenon of body weight increase can elevate the risk of cardiovascular disease and may also cause a side effect of deteriorating the function of controlling blood glucose levels.

Glucagon is produced by the pancreas when blood glucose levels drop as a result of other medications or diseases, or hormone or enzyme deficiencies. Glucagon sends a signal for glycogen breakdown in the liver and a subsequent glucose release and has a role in raising blood glucose levels to a normal range. In addition to the effect of increasing the blood glucose levels, it was reported that glucagon suppresses appetite and activates hormone-sensitive lipase of adipocytes to facilitate lipolysis, thereby showing an anti-obesity effect.

One of the glucagon derivatives, glucagon-like peptide-1 (GLP-1), is under development as a therapeutic agent for treating hyperglycemia in patients with diabetes. GLP-1 has the functions of stimulating insulin synthesis and secretion, inhibiting glucagon secretion, slowing gastric emptying, increasing glucose utilization, and inhibiting food intake. Exendin-4, prepared from lizard venom and having an amino acid homology of about 50% with GLP-1, was also reported to activate the GLP-1 receptor, thereby ameliorating hyperglycemia in patients with diabetes. However, it was reported that anti-obesity drugs containing GLP-1 have problems in that they show side-effects such as vomiting and nausea.

Fibroblast growth factor 21 (FGF21) is a secretory polypeptide belonging to a subfamily of fibroblast growth factor (FGF) which includes FGF19, FGF21, and FGF23. FGF21 has an important role in various physiological functions including angiogenesis, mitotic reproduction, pattern formation, cell differentiation, control of metabolism, and recovery of tissue damage. Reportedly, FGF21 is mainly expressed in the liver and has been described as therapeutic agents for treating diseases, such as ischemic vascular disease, wound healing, and diseases associated with the functional damages in the lung, bronchi, or alveolar cells and other numerous disorders.

According to WO 2003/011213, when FGF21 is subjected to a chronic treatment (72 hours) in murine 3T3-L1 adipocytes in the presence or absence of insulin, FGF21 stimulates the glucose uptake in the adipocytes. Additionally, FGF21 was shown to reduce the levels of blood glucose, triglycerides, and glucagon in fasted and fed ob/ob mice, db/db mice, and ZDF rats (8 weeks old) in a dose-dependent manner. As such, the effect of FGF21 to be used as a therapy for the treatment of diabetes and obesity has been confirmed.

Meanwhile, the present inventors have previously developed a protein conjugate in which an insulin analog with an increased half-life compared to that of native type insulin and a physiologically active polypeptide such as imidazoacetyl (CA) exendin-4 (CA-exendin-4) were linked to an immunoglobulin Fc region through a non-peptide polymer by a site-specific covalent linkage, and a composition containing the same, respectively (WO 2014/133324 and WO 2008/082274). However, there has been no report disclosing the forms in which two or more kinds of physiologically active polypeptides are linked to a single unit of an immunoglobulin Fc region by a non-peptide polymer, respectively.

Under these circumstances, the present inventors have made efforts to develop a single material having two or more kinds of effects while reducing the amount of immunoglobulin Fc, which is administered double the dose in a case of combined administration or when used in the form of a complex formulation. As a result, they have developed a physiologically active polypeptide conjugate, which includes multiple physiologically active polypeptides and an immunoglobulin Fc region. Specifically, a heterodimer conjugate, which is in a form where each of two different kinds of polypeptides exhibiting their respective physiological activities is covalently linked to a single unit of an immunoglobulin Fc, which is in a dimeric form, by a non-peptide polymer, was developed.

When the *in-vitro* titer of the heterodimer conjugate was compared to that of a monomer conjugate, in which one kind of a physiological material covalently linked to a single unit of an immunoglobulin Fc by a non-peptide polymer, it was confirmed that the level of the corresponding physiological activity of the heterodimer conjugate was equivalent to that of the monomer conjugate. Accordingly, the present inventors have confirmed that when the heterodimer conjugate is administered to the human body, it can reduce the concentration of the immunoglobulin Fc administered to half compared to when two or more monomer conjugates are administered in combination or a complex formulation thereof is administered, and the concentration and amount of the protein(s) administered are also decreased, and thus the viscosity increase that may occur according to the protein concentration and the subsequent pain caused that patients must endure during injections can be alleviated, thereby completing the present invention.

### [Disclosure]

### [Technical Problem]

An object of the present invention is to provide a protein conjugate of a physiologically active polypeptide, which includes multiple physiologically active polypeptides and an immunoglobulin Fc region.

Specifically, the object of the present invention is to provide a protein conjugate including two or more physiologically active polypeptides, wherein the protein conjugate includes: (i) a unit of an immunoglobulin Fc region or a part of the Fc region having an FcRn-binding affinity; (ii) a non-peptide polymer covalently linked to the Fc region or the part thereof; and (iii) a physiologically active polypeptide covalently linked to the non-peptide polymer; wherein the Fc region or the part thereof is a dimer of two polypeptide chains, and one or more of the non-peptide polymers are linked to each of the two polypeptide chains.

Another object of the present invention is to provide a method for preparing the protein conjugate.

Still another object of the present invention is to provide a composition containing the protein conjugate.

Still another object of the present invention is to provide a composition for improving *in-vivo* duration and stability of physiologically active polypeptides, containing the protein conjugate.

Still another object of the present invention is to provide a method for treating a target disease, including administering the protein conjugate or a composition containing the protein conjugate to a subject in need thereof.

### [Technical Solution]

An aspect of the present invention provides a protein conjugate of physiologically active polypeptides including multiple physiologically active polypeptides and an immunoglobulin Fc region.

Specifically, in an aspect, the present invention provides a protein conjugate including two or more physiologically active polypeptides, wherein the protein conjugate includes: (i) a unit of an immunoglobulin Fc region or a part of the Fc region having an FcRn-binding affinity; (ii) a non-peptide polymer covalently linked to the Fc region or the part thereof; and (iii) a physiologically active polypeptide covalently linked to the non-peptide polymer; wherein the Fc region or the part thereof is a dimer of two polypeptide chains, and one or more of the non-peptide polymers are linked to each of the two polypeptide chains.

In a specific embodiment, the immunoglobulin Fc region is aglycosylated.

In another specific embodiment, the immunoglobulin Fc region is composed of one to four domains selected from the group consisting of a CH1 domain, a CH2 domain, a CH3 domain, and a CH4 domain.

In still another specific embodiment, the immunoglobulin Fc region further includes a hinge region.

In still another specific embodiment, the immunoglobulin Fc region is selected from the group consisting of Fc regions of IgG, IgA, IgD, IgE, IgM, a combination thereof, and a hybrid thereof.

In still another specific embodiment, the immunoglobulin Fc region is selected from the group consisting of Fc regions of IgG1, IgG2, IgG3, IgG4, a combination thereof, and a hybrid thereof.

In still another specific embodiment, the immunoglobulin Fc region is an IgG4 Fc region.

In still another specific embodiment, the immunoglobulin Fc region is a human aglycosylated IgG4 Fc region.

In still another specific embodiment, the covalent linkage is formed by reacting a reactive group at one end of a non-peptide polymer selected from the group consisting of an aldehyde group, a maleimide group, and a succinimide derivative with an amino group of the Fc region or the part thereof or an amino group of the physiologically active polypeptide.

In still another specific embodiment, the succinimide derivative is succinimidyl propionate, succinimidyl carboxymethyl, hydroxy succinimidyl, or succinimidyl carbonate.

In still another specific embodiment, the reactive group at both ends of the non-peptide polymer is an aldehyde group.

In still another specific embodiment, the one end of the non-peptide polymer is linked to any one selected from a *N*-terminus, a side chain of a lysine residue, and a side chain of an arginine residue of the immunoglobulin Fc, and the other end of the non-peptide polymer is linked to any one selected from a *N*-terminus, a side chain of a lysine residue, a side chain of an arginine residue, a side chain of a histidine residue, and a side chain of a cysteine residue of the physiologically active polypeptide.

In still another specific embodiment, the non-peptide polymer is at least one kind selected from the group consisting of polyethylene glycol, polypropylene glycol, an ethylene glycol-polypropylene glycol copolymer, polyoxyethylated polyol, polyvinyl alcohol, a polysaccharide, dextran, polyvinyl ethyl ether, a biodegradable polymer, lipid polymer, fatty acid, chitin, hyaluronic acid, and a combination thereof.

In still another specific embodiment, the non-peptide polymer is polyethylene glycol.

In still another specific embodiment, the physiologically active polypeptide is selected from the group consisting of hormones, cytokines, enzymes, antibodies, growth factors, transcription factors, blood factors, vaccines, structural proteins, ligand proteins, and receptors.

In still another specific embodiment, the physiologically active polypeptide is selected from the group consisting of insulin, glucagon, glucagon-like peptides, oxyntomodulin, exendin-3, exendin-4, fibroblast growth factor 21 (FGF21), human growth hormone (hGH), growth hormone-releasing hormone, growth hormone-releasing peptides, interferons, interferon receptors, colony-stimulating factors, G protein-coupled receptors, interleukins, interleukin receptors, enzymes, interleukin-binding proteins, cytokine-binding proteins, macrophage-activating factors, macrophage peptides, B cell factor, T cell factor, protein A, allergy inhibitors, cell necrosis glycoprotein, immunotoxin, lymphotoxin, tumor necrosis factors, tumor inhibitory factors, metastasis-inducing factors, alpha-1-antitrypsin, albumin, α-lactalbumin, apolipoprotein-E, erythropoietin, highly glycosylated erythropoietin, angiopoietins, hemoglobin, thrombin, thrombin receptor-activating peptides, thrombomodulin, blood factors VII, VIIa, VIII, IX, and XIII, plasminogen-activating factors, fibrin-binding peptides, urokinase, streptokinase, hirudin, protein C, C-reactive protein, renin inhibitors, collagenase inhibitors, superoxide dismutase, leptin, platelet-derived growth factors, epidermal growth factors, epithelial cell growth factors, angiostatin, osteogenic growth factors, osteogenesis-promoting proteins, calcitonin, atriopeptin, cartilage-inducing factors, elcatonin, connective tissue-activating factors, tissue factor pathway inhibitors, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, glucagon, cholecystokinin, pancreatic polypeptides, gastrin-releasing peptides, corticotropin-releasing factor, thyroid-stimulating hormone, autotaxin, lactoferrin, myostatin, receptors, receptor antagonists, cell surface antigens, virus-derived vaccine antigens, monoclonal antibody, polyclonal antibody, antibody fragments, erythropoietic growth factors, leukopoietin, amylin, somatostatin, peptide YY (PYY), neuropeptide Y (NPY), angiotensin, bradykinin, calcitonin, corticotropin, eledoisin, gastrin, leptin, oxytocin, vasopressin, luteinizing hormone, luteotropin, follicle-stimulating hormone, parathyroid hormone, secretin, sermorelin, human growth hormone (hGH), growth hormone-releasing peptides, granulocyte colony-stimulating factors (GCSF), interferons (IFN), interleukins, prolactin-releasing peptides, orexin, thyroid-releasing peptides, cholecystokinin, gastrin inhibitory peptides, calmodulin, gastric-releasing peptides, motilin, vasoactive intestinal peptides, atrial natriuretic peptides (ANPs), barin natriuretic peptides (BNPs), C-type natriuretic peptides (CNPs), neurokinin A, neuromedin, renin, endothelin, sarafotoxin peptide, carsomorphin peptide, dermorphin, dynorphin, endorphin, enkepalin, T cell factor, tumor necrosis factors, tumor necrosis factor receptors, urokinase receptors, tumor inhibitory factors, collagenase inhibitors, thymopoietin, thymulin, thymopentin, thymosin, thymic humoral factor, adrenomedullin, allatostatin, amyloid beta-protein fragments, antibacterial peptides, antioxidant peptides, bombesin, osteocalcin, CART peptides, E-selectin, ICAM-1, VCAM-1, leucokine, kringle-5, laminin, inhibin, galanin, fibronectin, pancreastatin, fuzeon, and analogs thereof.

In still another specific embodiment, the physiologically active polypeptide is selected from the group consisting of insulin, glucagon, glucagon-like peptides, oxyntomodulin, exendin-3, exendin-4, fibroblast growth factor 21 (FGF21), human growth hormone, interferon-alpha, granulocyte colony-stimulating factors (G-CSF), erythropoietin, Fab' antibody fragments, and an analog thereof.

In still another specific embodiment, the physiologically active polypeptides linked to each of the two polypeptide chains of the Fc region or a part thereof differ from each other.

In still another specific embodiment, each of two units of the physiologically active polypeptides is linked to a single unit of the immunoglobulin Fc region or a part thereof by a non-peptide polymer, and the kinds of the two units are different from each other.

In still another specific embodiment, physiologically active polypeptides of different kinds are linked to a single unit of the immunoglobulin Fc region or a part thereof by a non-peptide polymer, respectively, and the physiologically active polypeptides of different kinds are present in a different ratio.

In still another specific embodiment, the physiologically active polypeptide, which is covalently linked to the immunoglobulin Fc region or a part thereof by a non-peptide polymer, is further linked with another physiologically active polypeptide, in a site other than the site to which the non-peptide polymer is linked.

In still another specific embodiment, each of three or more units of physiologically active polypeptides is linked to a single unit of the immunoglobulin Fc region or a part thereof by a non-peptide polymer, and the kinds of the three or more units are different from each other.

To achieve the present invention, in another aspect, there is provided a homomultimer conjugate of physiologically active polypeptides, in which each of three or more units of physiologically active polypeptides is covalently linked to a single unit of the immunoglobulin Fc region or the part thereof having the FcRn-binding affinity, by a non-peptide polymer and wherein the three or more units exhibit the same physiological activity.

To achieve the present invention, in still another aspect, there is provided a method for preparing the protein conjugate.

Specifically, the method includes providing (a) a non-peptide polymer, which is linked at one end to a physiologically active polypeptide and includes a remaining reactive group at the other end, and (b) a physiologically active polypeptide conjugate, in which one or more physiologically active polypeptides are respectively linked to a unit of the immunoglobulin Fc region or a part thereof having the FcRn-binding affinity, wherein the unit of the immunoglobulin Fc region or a part thereof having the FcRn-binding affinity includes a remaining reactive group; and covalently linking the remaining reactive group of the non-peptide polymer, which is linked to the physiologically active polypeptide of (a), and the remaining reactive group of the immunoglobulin Fc region or a part thereof of the physiologically active polypeptide conjugate of (b).

In a specific embodiment, the non-peptide polymer of (a) includes a remaining reactive group selected from the group consisting of an aldehyde group, a maleimide group, and a succinimide derivative.

In another specific embodiment, the remaining reactive group of the immunoglobulin Fc regionor a part thereof of (b) is positioned in any of a *N*-terminus, a side chain of a lysine residue, and a side chain of an arginine residue of an immunoglobulin Fc.

In still another specific embodiment, the physiologically active polypeptide of (a) and the physiologically active polypeptide of (b) are of the same or different kinds with each other.

In still another specific embodiment, the physiologically active polypeptide of (a) and the physiologically active polypeptide of (b) are in a single unit.

To achieve the present invention, in still another aspect, there is provided a composition containing the protein conjugate.

In a specific embodiment, the composition is for improving *in-vivo* duration and stability of physiologically active polypeptides.

In another specific embodiment, the composition is a pharmaceutical composition.

To achieve the present invention, in still another aspect, there is provided a method for treating a target disease, including administering the protein conjugate or a composition containing the protein conjugate to a subject in need thereof.

### [Advantageous Effects of the Invention]

The conjugate of the present invention, in which two or more physiologically active polypeptides are linked to an immunoglobulin Fc by a non-peptide polymer, can have a physiological activity the same as or similar to each of the monomer conjugates, in which a single physiologically active polypeptide is linked to an immunoglobulin Fc by a non-peptide polymer. Therefore, the conjugate of the present invention can reduce the unnecessary amount of immunoglobulin Fc administration for the human body, compared to when a monomer conjugate is used.

Additionally, in a case of the conjugate of the present invention which includes different kinds of physiologically active polypeptides, the conjugate can further have the advantage of having two or more pharmaceutical effects in a single conjugate.

### [BRIEF DESCRIPTION OF THE DRAWINGS]

FIG. 1 shows the HPLC analysis results of a heterodimer conjugate including an insulin analog, imidazo-acetyl (CA) exendin-4, and an immunoglobulin Fc, wherein the long-acting insulin analog conjugate represents a monomer conjugate, in which an insulin analog is linked to an immunoglobulin Fc by PEG, and the long-acting CA-exendin-4 conjugate represents a monomer conjugate, in which CA-exendin-4 is linked to an immunoglobulin Fc by PEG.
FIG. 2 shows the SDS-PAGE (12%) results of a heterodimer conjugate including an insulin analog, imidazo-acetyl exendin-4, and an immunoglobulin Fc.
FIG. 3 shows the western blot analysis results of a heterodimer conjugate including an insulin analog, imidazo-acetyl exendin-4, and an immunoglobulin Fc, using an anti-immunoglobulin Fc.
FIG. 4 shows the western blot analysis results of a heterodimer conjugate including an insulin analog, imidazo-acetyl exendin-4, and an immunoglobulin Fc, using anti-exendin-4.
FIG. 5 shows the western blot analysis results of a heterodimer conjugate including an insulin analog, imidazo-acetyl exendin-4, and an immunoglobulin Fc, using anti-insulin.
FIG. 6 shows the HPLC analysis results of a homodimer conjugate including two units of an insulin analog and an immunoglobulin Fc, wherein the long-acting insulin analog conjugate represents a monomer conjugate, in which an insulin analog is linked to an immunoglobulin Fc by PEG.
FIG. 7 shows the SDS-PAGE analysis results of a homodimer conjugate including two units of an insulin analog and an immunoglobulin Fc.
FIG. 8 shows the HPLC analysis results of a homodimer conjugate including two units of imidazo-acetyl exendin-4 and an immunoglobulin Fc.
FIG. 9 shows the SDS-PAGE analysis results of a homodimer conjugate including two units of imidazo-acetyl exendin-4 and an immunoglobulin Fc.
FIG. 10 shows the HPLC analysis results of a heterodimer conjugate including an insulin analog, a glucagon analog, and an immunoglobulin Fc.
FIG. 11 shows the SDS-PAGE analysis results of a heterodimer conjugate including an insulin analog, a glucagon analog, and an immunoglobulin Fc.
FIG. 12 shows the HPLC analysis results of a heterodimer conjugate including imidazo-acetyl exendin-4, a glucagon analog, and an immunoglobulin Fc.
FIG. 13 shows the SDS-PAGE analysis results of a heterodimer conjugate including imidazo-acetyl exendin-4, a glucagon analog, and an immunoglobulin Fc.
FIG. 14 shows the HPLC analysis results of a heterodimer conjugate including imidazo-acetyl exendin-4, fibroblast growth factor 21, and an immunoglobulin Fc.
FIG. 15 shows the SDS-PAGE analysis results of a heterodimer conjugate including imidazo-acetyl exendin-4, fibroblast growth factor 21, and an immunoglobulin Fc.

### [Detailed Description of the Invention]

Hereinbelow, specific embodiments of the present invention will be described in detail. Meanwhile, each of the explanations and specific embodiments disclosed herein may be applied to other explanations and specific embodiments. That is, all combinations of various factors disclosed herein belong to the scope of the present invention. Furthermore, the scope of the present invention should not be limited by the specific disclosure provided herein below.

To achieve the present invention, in an aspect, there is provided a protein conjugate of physiologically active polypeptides, which includes multiple physiologically active polypeptides and an immunoglobulin Fc region.

Specifically, the present invention provides a protein conjugate including two or more physiologically active polypeptides, wherein the protein conjugate includes: (i) a unit of an immunoglobulin Fc region or a part of the Fc region having an FcRn-binding affinity; (ii) a non-peptide polymer covalently linked to the Fc region or the part thereof; and (iii) a physiologically active polypeptide covalently linked to the non-peptide polymer; wherein the Fc region or the part thereof is a dimer of two polypeptide chains, and one or more of the non-peptide polymers are linked to each of the two polypeptide chains.

As used herein, the term "protein conjugate" refers to a conjugate which includes a physiologically active polypeptide and any different component, and specifically, a material in which a physiologically active polypeptide and any different component are covalently linked.

The any different component may be any material which has any advantageous function with respect to *in-vivo* actions of physiologically active polypeptides, such as increasing the blood half-life of physiologically active polypeptides or reducing their renal clearance.

The any different component may include a carrier. Examples of the carrier that can constitute the protein conjugate of the present invention may include albumin, transferrin, antibody, antibody fragments, elastin, heparin, a polysaccharide such as chitin, and fibronectin which can be linked to physiologically active polypeptides, thereby increasing stability in the blood, but is not limited thereto.

In a specific embodiment of the present invention, the different component may be an immunoglobulin Fc region or a part of the immunoglobulin Fc region having a binding affinity for a neonatal Fc receptor (FcRn), but is not limited thereto.

The immunoglobulin Fc region or a part thereof may be in a dimeric form of two polypeptide chains. In particular, the two polypeptide chains may have a dimeric form by any linkage including a disulfide bond, etc. Additionally, the part of the immunoglobulin Fc region that can be used for a protein conjugate may be a part of the Fc region that can bind to FcRn.

More specifically, the protein conjugate may be in a form in which two or more physiologically active polypeptides are linked to a single unit of an immunoglobulin Fc region or a part thereof by a non-peptide polymer, but is not limited thereto.

In a specific embodiment of the present invention, the protein conjugate may be one in which a physiologically active polypeptide is linked per each of the immunoglobulin heavy chain, that constitutes an immunoglobulin Fc region in a dimeric form or a part of the Fc region having the FcRn binding affinity by a non-peptide polymer, but is not limited thereto.

The non-peptide polymer can be interposed between a physiologically active polypeptide and an immunoglobulin Fc region or a part thereof, and specifically, between the physiologically active polypeptide and the immunoglobulin heavy chain constituting the Fc region or a part thereof, thereby connecting the two by a covalent linkage.

The protein conjugate may be in the form of a homomultimer or heteromultimer, more specifically, a homodimer or homotrimer or heterodimer or heterotrimer, and even more specifically, a homodimer or heterodimer.

As used herein, the term "homomultimer" refers to a conjugate, which is in a form where each of the two or more polypeptide units showing the same physiological activity is linked to a single unit of an immunoglobulin Fc region or a part thereof by a non-peptide polymer.

That is, the physiologically active polypeptides, which are linked to a single unit of an immunoglobulin Fc region or a part thereof show the same physiological activity, and thus the conjugate can show a single physiological activity.

In the present invention, the term "homomultimer" may be used interchangeably with "homomultimer conjugate".

As used herein, the term "homodimer" refers to a conjugate, which is in a form where each of the two polypeptide units showing the same physiological activity is linked to a single unit of an immunoglobulin Fc region or a part thereof by a non-peptide polymer.

Specifically, the homodimer may have a structure, wherein each of two polypeptide units showing the same physiological activity is linked via a non-peptide polymer to each of the two polypeptide chains in a single unit of an immunoglobulin Fc region or a part thereof.

In the present invention, the term "homodimer" may be used interchangeably with "homodimer conjugate".

As used herein, the term "homotrimer" refers to a conjugate, which is in a form where each of the three polypeptide units showing the same physiological activity is linked to a single unit of an immunoglobulin Fc region or a part thereof by a non-peptide polymer, respectively.

For example, the homotrimer may have a structure, in which one non-peptide polymer and two non-peptide polymers are linked to each of the two polypeptide chains of a single unit of an immunoglobulin Fc region or a part thereof, respectively, and each of the three physiologically active polypeptide units is linked to the corresponding non-peptide polymer, respectively, but is not particularly limited thereto.

In the present invention, the term "homotrimer" may be used interchangeably with "homotrimer conjugate".

As used herein, the term "heteromultimer" refers to a conjugate, which is in a form where each of the two or more polypeptide units (*e.g*., two units or three units or more) showing a different physiological activity is linked to a single unit of an immunoglobulin Fc region or a part thereof by a non-peptide polymer.

Accordingly, the heteromultimer conjugate may include two or more types of physiologically active polypeptides, thereby showing dual or multiple physiological activities.

For example, heteromultimer may have a structure, in which one or two or more of non-peptide polymers are linked to each of the two polypeptide chains of a single unit of an immunoglobulin Fc region or a part thereof, and each of the physiologically active polypeptides are linked to the corresponding non-peptide polymer, but is not particularly limited thereto.

In the present invention, the term "heteromultimer" may be used interchangeably with "heteromultimer conjugate".

The heteromultimer may be in the form of a heterodimer, a heterotrimer, etc., but is not particularly limited thereto.

As used herein, the term "heterodimer" refers to a conjugate, which is in a form where each of the two polypeptide units showing a different physiological activity is linked to a single unit of an immunoglobulin Fc region or a part thereof by a non-peptide polymer.

Specifically, the heterodimer may have a structure, in which each of the two physiological polypeptide units showing a different physiological activity is linked via a non-peptide polymer to each of the two polypeptide chains of a single unit of an immunoglobulin Fc region or a part thereof.

In the present invention, the term "heterodimer" may be used interchangeably with "heterodimer conjugate".

As used herein, the term "heterotrimer" refers to a conjugate, which is in a form where each of the three polypeptide units showing a different physiological activity is linked to a single unit of an immunoglobulin Fc region or a part thereof by a non-peptide polymer.

For example, heterotrimer may have a structure, in which one non-peptide polymer and two non-peptide polymers are linked to each of the two polypeptide chains of a single unit of an immunoglobulin Fc region or a part thereof, and each of the three units of physiologically active polypeptides is linked to the corresponding non-peptide polymer, respectively, but is not particularly limited thereto.

In the present invention, the term "heterotrimer" may be used interchangeably with "heterotrimer conjugate".

In particular, the physiologically active polypeptide, which is covalently linked via a non-peptide polymer to an immunoglobulin Fc region or a part thereof, constituting the homomultimer or heteromultimer conjugate may be further linked with another physiologically active polypeptide, in a site other than the site to which the non-peptide polymer is linked, but is not particularly limited thereto.

In particular, the additional physiologically active polypeptides may be in a form, in which two or more physiologically active polypeptides are linked via a linker, or directly linked to each other, but is not limited thereto. The linker may be a peptide linker or non-peptide linker, but is not limited thereto.

A form, in which at least one or at least one kind of a physiologically active polypeptide is continuously linked by a linker on any region of a physiologically active polypeptide linked via a non-peptide polymer to a polypeptide chain of a single unit of an immunoglobulin Fc, is also included.

In particular, the physiologically active polypeptide to be added additionally may be the same kind of a physiologically active polypeptide as the physiologically active polypeptide linked thereto, but is not limited thereto. In this case, all kinds or some kinds of the physiologically active polypeptides constituting the homomultimer and the physiologically active polypeptides constituting the heteromultimer may be present in the form of multiple polypeptides. That is, they may have a structure in which a physiologically active polypeptide, in the form of multiple polypeptides, is linked to an immunoglobulin Fc region by a non-peptide polymer, but is not particularly limited thereto.

Additionally, in the heteromultimer, different kinds of physiologically active polypeptides may be present in a different ratio from each other.

For example, two different kinds of physiologically active polypeptides can be present in a different ratio from each other, by linking two different kinds of physiologically active polypeptides, which are linked to each of the two physiologically active polypeptide chains constituting a single unit of an immunoglobulin Fc or a part thereof by a non-peptide polymer, in which one of the two kinds of the physiologically active polypeptides is further linked to a physiologically active polypeptide of the same kind, but the ratio is not limited thereto.

In a specific embodiment, the protein conjugate is a heterodimer, in which a single unit of an insulin analog and a single unit of imidazo-acetyl exendin-4 (*i.e.,* an insulinotropic peptide) are linked to each of the polypeptides of a single unit of an Fc region by a non-peptide polymer, respectively.

In a specific embodiment of the present invention, the protein conjugate is a heterodimer, in which a single unit of an insulin analog and a single unit of a glucagon analog are linked to each of the polypeptides of a single unit of an Fc region by a non-peptide polymer, respectively.

In a specific embodiment, the protein conjugate is a heterodimer, in which a single unit of imidazo-acetyl exendin-4 (*i.e.,* an insulinotropic peptide) and a single unit of a glucagon analog are linked to each of the polypeptides of a single unit of an Fc region by a non-peptide polymer, respectively.

In a specific embodiment, the protein conjugate is a heterodimer, in which a single unit of imidazo-acetyl exendin-4 (*i.e.,* an insulinotropic peptide) and a single unit of FGF21 are linked to each of the polypeptides of a single unit of an Fc region by a non-peptide polymer, respectively.

The heteromultimer described above, in particular the heteromultimer, may exhibit a physiological activity which is equal or corresponding to a monomer conjugate of a physiologically active polypeptide constituting the heteromultimer, but is not particularly limited thereto.

As used herein, the term "monomer" refers to a conjugate in a form where a single unit of a polypeptide exhibiting a physiological activity is linked to a single unit of an immunoglobulin Fc region by a non-peptide polymer.

Specifically, the monomer refers to a conjugate in a form where a single unit of a physiologically active polypeptide is linked to any one polypeptide chain of the two polypeptide chains of a single unit of an immunoglobulin Fc region by a non-peptide polymer.

In the present invention, "monomer" may be used interchangeably with "monomer conjugate".

The physiologically active polypeptide, which is a moiety constituting the protein conjugate, is a material that enables to exhibit a physiological activity in the human body, and any peptide which can exhibit physiological activity may be included.

For example, the physiologically active polypeptide may be selected from the group consisting of hormones, cytokines, enzymes, antibodies, growth factors, transcription factors, blood factors, vaccines, structural proteins, ligand proteins, and receptors.

Additionally, for example, the physiologically active polypeptide may be selected from the group consisting of insulinotropic peptide, blood factors, digestion-promoting hormone, adrenocortical hormone, thyroid hormone, intestinal hormones, cytokines, enzymes, growth factors, neuropeptides, pituitary hormone, hypothalamus hormone, anti-obesity peptide, anti-virus peptide, non-natural peptide derivatives having physiological activities, and analogs thereof, but is not limited thereto. Additionally, the physiologically active polypeptide may be a GLP-1 receptor agonist, a leptin receptor agonist, a DPP-IV inhibitor, a Y5 receptor antagonist, a melanin-concentrating hormone (MCH) receptor antagonist, a Y2/3 receptor agonist, an MC3/4 receptor agonist, a gastric/pancreatic lipase inhibitor, a 5HT2c agonist, a β3A receptor agonist, an amylin receptor agonist, a ghrelin antagonist, or a ghrelin receptor antagonist.

More specifically, the physiologically active polypeptide may be one selected from the group consisting of insulin, glucagon, glucagon-like peptides, oxyntomodulin, exendin-3, exendin-4, fibroblast growth factor 21 (FGF21), human growth hormone (hGH), growth hormone-releasing hormone, growth hormone-releasing peptides, interferons, interferon receptors, colony-stimulating factors, G protein-coupled receptors, interleukins, interleukin receptors, enzymes, interleukin-binding proteins, cytokine-binding proteins, macrophage-activating factors, macrophage peptides, B cell factor, T cell factor, protein A, allergy inhibitors, cell necrosis glycoprotein, immunotoxin, lymphotoxin, tumor necrosis factors, tumor inhibitory factors, metastasis-inducing factors, alpha-1-antitrypsin, albumin, α-lactalbumin, apolipoprotein-E, erythropoietin, highly glycosylated erythropoietin, angiopoietins, hemoglobin, thrombin, thrombin receptor-activating peptides, thrombomodulin, blood factors VII, VIIa, VIII, IX, and XIII, plasminogen-activating factors, fibrin-binding peptides, urokinase, streptokinase, hirudin, protein C, C-reactive protein, renin inhibitors, collagenase inhibitors, superoxide dismutase, leptin, platelet-derived growth factors, epidermal growth factors, epithelial cell growth factors, angiostatin, osteogenic growth factors, osteogenesis-promoting proteins, calcitonin, atriopeptin, cartilage-inducing factors, elcatonin, connective tissue-activating factors, tissue factor pathway inhibitors, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, glucagon, cholecystokinin, pancreatic polypeptides, gastrin-releasing peptides, corticotropin-releasing factor, thyroid-stimulating hormone, autotaxin, lactoferrin, myostatin, receptors, receptor antagonists, cell surface antigens, virus-derived vaccine antigens, monoclonal antibody, polyclonal antibody, antibody fragments, erythropoietic growth factors, leukopoietin, amylin, somatostatin, peptide YY (PYY), neuropeptide Y (NPY), angiotensin, bradykinin, calcitonin, corticotropin, eledoisin, gastrin, leptin, oxytocin, vasopressin, luteinizing hormone, luteotropin, follicle-stimulating hormone, parathyroid hormone, secretin, sermorelin, human growth hormone (hGH), growth hormone-releasing peptides, granulocyte colony-stimulating factors (GCSF), interferons (IFN), interleukins, prolactin-releasing peptides, orexin, thyroid-releasing peptides, cholecystokinin, gastrin inhibitory peptides, calmodulin, gastric-releasing peptides, motilin, vasoactive intestinal peptides, atrial natriuretic peptides (ANPs), barin natriuretic peptides (BNPs), C-type natriuretic peptides (CNPs), neurokinin A, neuromedin, renin, endothelin, sarafotoxin peptide, carsomorphin peptide, dermorphin, dynorphin, endorphin, enkepalin, T cell factor, tumor necrosis factors, tumor necrosis factor receptors, urokinase receptors, tumor inhibitory factors, collagenase inhibitors, thymopoietin, thymulin, thymopentin, thymosin, thymic humoral factor, adrenomedullin, allatostatin, amyloid beta-protein fragments, antibacterial peptides, antioxidant peptides, bombesin, osteocalcin, CART peptides, E-selectin, ICAM-1, VCAM-1, leucokine, kringle-5, laminin, inhibin, galanin, fibronectin, pancreastatin, fuzeon, and analogs thereof.

According to a specific embodiment of the present invention, 1) a combination of insulin or an analog thereof and glucagon or an analog thereof; 2) a combination of insulin or an analog thereof and an insulinotropic peptide or an analog thereof; 3) a combination of insulinotropic peptide or an analog thereof and glucagon or an analog thereof; and 4) a combination of insulinotropic peptide or an analog thereof and FGF21 or an analog thereof may be applicable to heteromultimers, but are not particularly limited thereto.

The physiologically active polypeptides not only include native physiologically active polypeptides but also analogs thereof.

In particular, the analogs may be the precursors-, derivatives-, and fragments of the native polypeptides having the physiological activities of the corresponding native polypeptides. They may be either naturally occurring or non-naturally occurring.

As used herein, the term "derivative of native polypeptide" includes a peptide which has at least one difference in an amino acid sequence compared to a native polypeptide; a peptide which is modified by modification of a native polypeptide sequence; and a mimic of a native polypeptide having the same physiological activity as the native polypeptide.

Specifically, derivatives of native polypeptide can be prepared by modifying a part of the amino acid(s) of the native polypeptide using any one of the methods of substitution, addition, deletion, and modification, or a combination thereof Additionally, the modification for the preparation of the derivatives of native polypeptide may include both a modification using an L-type or D-type amino acid(s), and/or non-natural type amino acid(s); and/or a modification of a native sequence (*e.g*., modification of a side chain reactive group, an intramolecular covalent bonding, such as a ring formation between side chains, methylation, acylation, ubiquitination, phosphorylation, amino-hexanation, biotinylation, etc.

Additionally, the modification may include the addition of one or more amino acids to an amino and/or carboxy end of a native polypeptide.

The amino acid(s) to be used for substitution or addition may include not only the 20 amino acids conventionally observed in human proteins but also atypical or non-naturally occurring amino acids. Commercial sources of the atypical amino acids include Sigma-Aldrich, ChemPep Inc., and Genzyme Pharmaceuticals. The peptides including these amino acids and typical peptide sequences may be synthesized and purchased from commercial suppliers, *e.g.,* American Peptide Company or Bachem (USA) or Anygen (Korea).

As used herein, the term "fragment of a native polypeptide or a derivative of a native polypeptide" refers to a form in which one or more amino acids are deleted to an amino and/or carboxy end of a native polypeptide or a derivative of a native polypeptide. Such a fragment of a native polypeptide may be possessed to the extent capable of exhibiting the same or corresponding physiological activity of the native polypeptide.

With respect to the information on insulin and examples of insulin analogs, the entire specifications of WO 2014/133324 and the corresponding US Patent Application Publication No. 2016-0008483 A1 are incorporated herein as references of the present invention.

Such insulin analogs may be those having a modification in the A-chain or B-chain, for example, a substitution of an amino acid selected from the group consisting of amino acids at positions 8, 23, 24, and 25 of the B-chain and amino acids at positions 1, 2, 14, and 19 of the A-chain with alanine, and specifically a substitution of an amino acid selected from the group consisting of amino acids at positions 8, 23, 24, and 25 of the B-chain and amino acids at positions 1, 2, and 19 with alanine; or a modification of an amino acid at position 14 of the A-chain with glutamic acid or asparagine, but is not limited thereto, and any insulin analog which possesses the function of controlling *in-vivo* blood glucose levels like insulin may be included in the scope of the present invention without limitation.

Additionally, in a specific embodiment, the insulin analog may be a peptide which includes the A-chain of SEQ ID NO: 68 represented by General Formula 1 below and the B-chain of SEQ ID NO: 69 represented by General Formula 2 below:

### [General Formula 1]

Xaa1-Ile-Val-Glu-Xaa2-Cys-Cys-Thr-Ser-Ile-Cys-Xaa3-Leu-Xaa4-Gln-Xaa5-Glu-A
sn-Xaa6-Cys-Xaa7 (SEQ ID NO: 68)

Wherein, in General Formula 1,
Xaa1 is alanine, glycine, glutamine, histidine, glutamic acid, or asparagine,
Xaa2 is alanine, glutamic acid, glutamine, histidine, or asparagine,
Xaa3 is alanine, serine, glutamine, glutamic acid, histidine, or asparagine,
Xaa4 is alanine, tyrosine, glutamic acid, histidine, lysine, aspartic acid or asparagine, Xaa5 is alanine, leucine, tyrosine, histidine, glutamic acid, or asparagine,
Xaa6 is alanine, tyrosine, serine, glutamic acid, histidine, or asparagine,
Xaa7 is asparagine, glycine, histidine, or alanine; and

### [General Formula 2]

Phe-Val-Asn-Gln-His-Leu-Cys-Gly-Ser-His-Leu-Val-Glu-Ala-Leu-Xaa8-Leu-Val-C
ys-Gly-Glu-Arg-Gly-Phe-Xaa9-Tyr-Xaa10-Xaa11-Lys-Thr (SEQ ID NO: 69)

in General Formula 2,
Xaa8 is tyrosine, glutamic acid, or aspartic acid, or absent,
Xaa9 is phenylalanine or absent,
Xaa10 is threonine or absent,
Xaa11 is proline, glutamic acid, or aspartic acid, or absent,
with the proviso that the sequence of native insulin may be excluded.

In a more specific embodiment, the insulin analog may be an insulin analog peptide, wherein, in General Formula 1 above, Xaa1 is glycine, Xaa2 is glutamine, Xaa3 is serine, Xaa4 is alanine, glutamic acid, or asparagine, Xaa5 is leucine, Xaa6 is tyrosine, Xaa7 is asparagine; and in General Formula 2 above, Xaa8 is tyrosine or glutamic acid, Xaa9 is phenylalanine or absent, Xaa10 is threonine, Xaa11 is proline, glutamic acid, or aspartic acid, or absent, but is not limited thereto.

In another more specific embodiment, the insulin analog may be an insulin analog peptide, wherein, in General Formula 1 above, Xaa1 is glycine, Xaa2 is glutamine, Xaa3 is serine, Xaa4 is glutamic acid or asparagine, Xaa5 is leucine, Xaa6 is tyrosine, Xaa7 is asparagine, in General Formula 2 above, Xaa8 is tyrosine, Xaa9 is phenylalanine or absent, Xaa10 is threonine, Xaa11 is proline, glutamic acid, or aspartic acid, or absent, but is not limited thereto.

In still another more specific embodiment, the insulin analog may be a peptide which includes the A-chain of General Formula 1 above and the B-chain of SEQ ID NO: 69, wherein in the B-chain, Xaa8 is tyrosine, Xaa9 is absent, Xaa10 is threonine, Xaa11 is proline, glutamic acid, or aspartic acid, or absent, but is not limited thereto.

The peptide may be one which includes the A-chain of General Formula 1 above and the B-chain of SEQ ID NO: 69, wherein in the B-chain, Xaa8 is tyrosine, Xaa9 is phenylalanine, Xaa10 is absent, Xaa11 is proline, glutamic acid, or aspartic acid, or absent, but is not limited thereto.

The peptide may be one, wherein, in the A-chain of SEQ ID NO: 68 above, Xaa1 is glycine, Xaa2 is glutamine, Xaa3 is serine, Xaa4 is glutamic acid, Xaa5 is leucine, Xaa6 is tyrosine, and Xaa7 is asparagine; and in the B-chain of SEQ ID NO: 69, Xaa8 is tyrosine, Xaa9 is phenylalanine, Xaa10 is threonine, Xaa11 is proline, glutamic acid, or aspartic acid, or absent, but is not limited thereto.

The peptide may be one, wherein in the A-chain of SEQ ID NO: 68 above, Xaa1 is glycine, Xaa2 is glutamine, Xaa3 is serine, Xaa4 is asparagine, Xaa5 is leucine, Xaa6 is tyrosine, and Xaa7 is asparagine; and in the B-chain of SEQ ID NO: 69, Xaa8 is tyrosine, Xaa9 is phenylalanine, Xaa10 is threonine, Xaa11 is proline, glutamic acid, or aspartic acid, or absent, but is not limited thereto.

In still another more specific embodiment, the insulin analog may be a peptide, wherein, in the A-chain of SEQ ID NO: 68 above, Xaa1 is glycine, Xaa2 is glutamine, Xaa3 is serine, Xaa4 is glutamic acid, Xaa5 is leucine, Xaa6 is tyrosine, Xaa7 is asparagine; and in the B-chain of SEQ ID NO: 69, Xaa8 is tyrosine, Xaa9 is absent, Xaa10 is threonine, Xaa11 is proline, glutamic acid, or aspartic acid, or absent, but is not limited thereto.

In still another more specific embodiment, the insulin analog may be a peptide, wherein, in the A-chain of SEQ ID NO: 68 above, Xaa1 is glycine, Xaa2 is glutamine, Xaa3 is serine, Xaa4 is alanine, Xaa5 is leucine, Xaa6 is tyrosine, Xaa7 is asparagine; and in the B-chain of SEQ ID NO: 69, Xaa8 is glutamic acid, Xaa9 is absent, Xaa10 is threonine, Xaa11 is proline, glutamic acid, or aspartic acid, or absent, but is not limited thereto.

Additionally, the insulin analog may include those peptides, in which the amino acid at position 14 of the A-chain of native insulin was modified to glutamic acid, asparagine, or alanine; and/or the amino acid at position 25 of the B-chain of native insulin was deleted; and/or the amino acid at position 16 (*i.e*., tyrosine) of the B-chain of native insulin was modified to glutamic acid, but is not limited thereto.

As examples of insulinotropic peptides and derivatives thereof, the entire specifications of WO 2008/082274 and the corresponding US Patent Application Publication No. 2010-0105877 A1 are incorporated herein as references of the present invention.

For example, an insulinotropic peptide is a peptide having the function of insulin secretion, and it can stimulate the synthesis and expression of insulin in the pancreatic beta cell. Examples of the insulinotropic peptide may include glucagon-like peptide (GLP)-1, exendin-3, exendin 4, etc., but is not limited thereto.

The analogs of the insulinotropic peptide may be in a form in which the *N*-terminal amine group of the insulinotropic peptide is deleted; a form in which the *N*-terminal amine group of the insulinotropic peptide is substituted with a hydroxyl group; a form in which the *N*-terminal amine group of the insulinotropic peptide is modified with two methyl groups; or a form in which alpha carbon of histidine (*i.e*., the 1^{st} amino acid of the insulinotropic peptide) is removed, etc., but is not limited thereto.

Specific examples of exendin-4 analogs may include desamino-histidyl exendin-4, in which the *N*-terminal amine group of exendin-4 is removed; β-hydroxy-imidazopropionyl exendin-4, in which the *N*-terminal amine group of exendin-4 is substituted with a hydroxyl group; dimethylhistidyl-exendin-4, in which the *N*-terminal amine group of exendin-4 is modified with two methyl groups; and imidazoacetyl-exendin-4, in which the alpha carbon of histidine (*i.e*., the 1^{st} amino acid of exendin-4) is removed, etc., but is not limited thereto.

The glucagon analog may be a peptide including the amino acid sequence of General Formula 3 below, but are not limited thereto:
X1-X2-QGTF-X7-SD-X10-S-X12-X13-X14-X15-X16-X17-X18-X19-X20-X21-F-X 23-X24-W-L-X27-X28-X29-X30 (General Formula 3, SEQ ID NO: 45) wherein, in General Formula 3,
X1 is histidine, desamino-histidyl, *N*-dimethyl-histidyl, beta-hydroxy imidazopropionyl, 4-imidazoacetyl, beta-carboxy imidazopropionyl, tryptophan, or tyrosine, or absent;
X2 is α-methyl-glutamic acid, aminoisobutyric acid (Aib), D-alanine, glycine, Sar(*N*-methylglycine), serine, or D-serine;
X7 is threonine, valine, or cysteine;
X10 is tyrosine or cysteine;
X12 is lysine or cysteine;
X13 is tyrosine or cysteine;
X14 is leucine or cysteine;
X15 is aspartic acid, glutamic acid, or cysteine;
X16 is glutamic acid, aspartic acid, serine, α-methyl-glutamic acid, or cysteine, or absent;
X17 is aspartic acid, glutamine, glutamic acid, lysine, arginine, serine, cysteine, or valine, or absent;
X18 is alanine, aspartic acid, glutamic acid, arginine, valine, or cysteine, or absent;
X19 is alanine, arginine, serine, valine, or cysteine, or absent;
X20 is lysine, histidine, glutamine, aspartic acid, lysine, arginine, α-methyl-glutamic acid, or cysteine, or absent;
X21 is aspartic acid, glutamic acid, leucine, valine, or cysteine, or absent;
X23 is isoleucine, valine, or arginine, or absent;
X24 is valine, arginine, alanine, cysteine, glutamic acid, lysine, glutamine, α-methyl-glutamic acid, or leucine, or absent;
X27 is isoleucine, valine, alanine, lysine, methionine, glutamine, or arginine, or absent;
X28 is glutamine, lysine, asparagine, or arginine, or absent;
X29 is lysine, alanine, glycine, or threonine, or absent; and
X30 is cysteine or absent,
(with the proviso that when the sequence is identical to that of native glucagon, it is excluded).

Additionally, more specifically,
in General Formula 3 above,
X1 may be histidine, tryptophan, or tyrosine, or absent;
X2 may be serine or aminoisobutyric acid (Aib);
X7 may be threonine, valine, or cysteine;
X10 may be tyrosine or cysteine;
X12 may be lysine or cysteine;
X13 may be tyrosine or cysteine;
X14 may be leucine or cysteine;
X15 may be aspartic acid or cysteine;
X16 may be glutamic acid, serine, or cysteine;
X17 may be aspartic acid, glutamic acid, lysine, arginine, serine, cysteine, or valine;
X18 may be aspartic acid, glutamic acid, arginine, or cysteine;
X19 may be alanine or cysteine;
X20 may be glutamine, aspartic acid, lysine, or cysteine;
X21 may be aspartic acid, glutamic acid, leucine, valine, or cysteine;
X23 may be isoleucine, valine, or arginine;
X24 may be valine, arginine, alanine, glutamic acid, lysine, glutamine, or leucine;
X27 may be isoleucine, valine, alanine, methionine, glutamine, or arginine;
X28 may be glutamine, lysine, asparagine, or arginine;
X29 may be threonine; and
X30 may be cysteine or absent.

In a more specific embodiment, the glucagon analog may include the following kinds, but is not limited thereto. However, SEQ ID NO: 1 represents the sequence of native glucagon.

**[Table 1]**

| SEQ ID NO | Peptide Sequence |
|---|---|
| SEQ ID NO:: 1 | HSQGTFTSDYSKYLDSRRAQDFVQWLMNT |
| SEQ ID NO:: 2 | HSQGTFTSDYSKYLDCDRAQDFVQWLMNT |
| SEQ ID NO:: 3 | HSQGTFTSDYSKYLDCERAQDFVQWLMNT |
| SEQ ID NO:: 4 | HSQGTFTSDYSKYLDSCDAQDFVQWLMNT |
| SEQ ID NO:: 5 | HSQGTFTSDYSKYLDSCEAQDFVQWLMNT |
| SEQ ID NO:: 6 | HSQGTFTSDYSKYLDSCEADDFVQWLMNT |
| SEQ ID NO:: 7 | YSQGTFTSDYSKYLDSCEADDFVQWLMNT |
| SEQ ID NO:: 8 | YXQGTFTSDYSKYLDSCDAQDFVQWLINT |
| SEQ ID NO:: 9 | YXQGTFTSDYSKYLDSCDAQDFVVWLINT |
| SEQ ID NO:: 10 | YXQGTFTSDYSKYLDSCDADDFVVWLINT |
| SEQ ID NO:: 11 | YXQGTFTSDYSKYLDEKCAKEFVQWLMNT |
| SEQ ID NO:: 12 | YXQGTFTSDYSKYLD**E**KRA**K**EFVQWLMNTC |
| SEQ ID NO:: 13 | YXQGTFTSDYSCYLDSRRAQDFVQWLMNT |
| SEQ ID NO:: 14 | YXQGTFTSDYSKYLDCKRAKEFVQWLMNT |
| SEQ ID NO:: 15 | YXQGTFTSDYSKYLCEKRAQDFVVWLMNT |
| SEQ ID NO:: 16 | YXQGTFTSDYSKYLDCRRAQVFVQWLMRT |
| SEQ ID NO:: 17 | YXQGTFTSDYSKYLDCVRAQDFVQWLMRT |
| SEQ ID NO:: 18 | YXQGTFTSDYSKYLDSRRACDFRLWLMNT |
| SEQ ID NO:: 19 | YXQGTFTSDYSKYLC**E**KRA**K**EFVQWLMNT |
| SEQ ID NO:: 20 | YXQGTFTSDYSKYLD**E**CRA**K**EFVQWLMNT |
| SEQ ID NO:: 21 | YXQGTFTSDYSKYLD**E**KCA**K**EFVQWLMNT |
| SEQ ID NO:: 22 | YXQGTFTSDYSKYLD**E**KRC**K**EFVQWLMNT |
| SEQ ID NO:: 23 | YXQGTFTSDYSKYCD**E**KRA**K**EFVQWLMNT |
| SEQ ID NO:: 24 | YXQGTFTSDYSKCLD**E**KRA**K**EFVQWLMNT |
| SEQ ID NO:: 25 | YXQGTFTSDYSKYLD**E**KRA**K**CFVQWLMNT |
| SEQ ID NO:: 26 | WXQGTFTSDYSKYLD**E**CRA**K**DFVQWLMNT |
| SEQ ID NO:: 27 | YXQGTFVSDYSKYLD**E**CRA**K**DFVQWLMNT |
| SEQ ID NO:: 28 | WXQGTFVSDYSKYLD**E**CRA**K**DFVQWLMNT |
| SEQ ID NO:: 29 | YXQGTFTSDYSKCLD**E**RRA**K**DFVQWLMNT |
| SEQ ID NO:: 30 | WXQGTFTSDYSKCLD**E**RRA**K**DFVQWLMNT |
| SEQ ID NO:: 31 | YXQGTFTSDYSKYLDC**K**RAK**E**FVQWLMNT |
| SEQ ID NO:: 32 | -SQGTFTSDYSKYLD**E**CRA**K**EFVQWLMNT |
| SEQ ID NO:: 33 | YXQGTFTSDYSKYLDSRRAQDFVQWLMNT |
| SEQ ID NO:: 34 | WXQGTFTSDYSKYCD**E**RRA**K**EFVQWLMNT |
| SEQ ID NO:: 35 | YXQGTFTSDYSKYCD**E**RRA**K**EFVQWLMNT |
| SEQ ID NO:: 36 | YXQGTFTSDCSKYLD**E**RRA**K**EFVQWLMNT |
| SEQ ID NO:: 37 | YXQGTFTSDYSKYLD**E**RRA**K**EFVQWLMNTC |
| SEQ ID NO:: 38 | YXQGTFCSDYSKYLD**E**RRA**K**EFVQWLMNT |
| SEQ ID NO:: 39 | YXQGTFVSDCSKYLD**E**RRA**K**DFVQWLMNT |
| SEQ ID NO:: 40 | YXQGTFVSDYSKYLD**E**RRA**K**DFVQWLMNTC |
| SEQ ID NO:: 41 | YXQGTFCSDYSKYLD**E**RRA**K**DFVQWLMNT |
| SEQ ID NO:: 42 | YXQGTFCSDYSKYLDSRRAQDFVQWLMNT |
| SEQ ID NO:: 43 | YXQGTFTSDCSKYLDSRRAQDFVQWLMNT |
| SEQ ID NO:: 44 | YXQGTFTSDYSKYLDSRRAQDFVQWLMNTC |

| | |
|---|---|
| (In the above Table, the amino acids indicated in X represent a non-naturally-occurring amino acid aminoisobutyric acid (Aib) and those underlined represent a ring formation.) | |

The FGF21 analogs may include the followings.

The FGF21 analogs may be those which have a reduced binding affinity for FGF receptors (FGFR) or auxiliary receptors (beta-klotho) compared to the native fibroblast growth factor 21 (FGF21) and have one or more modified amino acids compared to the native FGF21, but are not limited thereto.

Specifically, the FGF21 analogs may be those which have a deletion(s) on the 1^{st} amino acid and/or 2^{nd} amino acid relative to the amino acid sequence of native FGF21, and furthermore, may include those, in which one or more amino acids selected from the amino acids at positions 167 and 168 are substituted with other amino acid(s) or modified, but are not limited thereto.

The FGF21 analogs may include deletion of the 1^{st} amino acid (*i.e*., histidine) and/or 2^{nd} amino acid (*i.e.,* proline) of the native FGF21, and independently or optionally further include substitution of the 167^{th} amino acid (*i.e*., serine) of native FGF21 with another amino acid, and independently or additionally include substitution of the 168^{th} amino acid (*i.e*., methionine) of native FGF21 with a different amino acid; or a combination of at least one of the substitutions thereof.

Further, the FGF21 analogs may include deletion of the 1^{st} amino acid (*i.e.,* histidine) of the *N*-terminus of native FGF21, and optionally further include the substitution of the 167^{th} amino acid (*i.e*., serine) of native FGF21 with other amino acid, substitution of the 168^{th} amino acid (*i.e.,* methionine) with another amino acid, or a combination of at least one of the substitutions thereof.

Additionally, the FGF21 analogs may include deletion of the 2^{nd} amino acid (*i.e*., proline) of the native FGF21, and optionally further include the substitution of the 167^{th} amino acid (*i.e*., serine) of native FGF21 with another amino acid, the substitution of the 168^{th} amino acid (*i.e.,* methionine) with another amino acid, or a combination of at least one of the substitutions thereof.

Furthermore, the FGF21 analogs may include deletion of the 1^{st} amino acid (*i.e.,* histidine) and the 2^{nd} amino acid (*i.e*., proline) of the native FGF21, and optionally further include the substitution of the 167^{th} amino acid (*i.e*., serine) of native FGF21 with another amino acid, the substitution of the 168^{th} amino acid (*i.e*., methionine) with another amino acid, or a combination of at least one of the substitutions thereof.

The FGF21 analogs may include the deletion of the 1^{st} amino acid (*i.e*., histidine) of the native FGF21, and further include the substitution of the 167^{th} amino acid (*i.e*., serine) or the 168^{th} amino acid (*i.e*., methionine) with threonine, alanine, phenylalanine, or isoleucine. Specifically, the FGF21 analogs may include the deletion of the 1^{st} amino acid (*i.e*., histidine) of the native FGF21, and further include the substitution of the 167^{th} amino acid (*i.e*., serine) with threonine, the substitution of the 168^{th} amino acid *(i.e.,* methionine) with alanine, phenylalanine, or isoleucine, or a combination of at least one of the substitutions thereof.

Additionally, the FGF21 analogs of the present invention may include the deletion of the 2^{nd} amino acid (*i.e.,* proline) of the native FGF21, and additionally include the substitution of the 167^{th} amino acid (*i.e*., serine) or the 168^{th} amino acid (*i.e*., methionine) of the native FGF21 with threonine, alanine, phenylalanine, or isoleucine. Specifically, the FGF21 analogs of the present invention may include the deletion of the 2^{nd} amino acid (*i.e*., proline) of the native FGF21, and further include the substitution of the 167^{th} amino acid (*i.e*., serine) with threonine, the subsitutuion of the 168^{th} amino acid (*i.e*., methionine) with alanine, phenylalanine, or isoleucine, or a combination of at least one of the substitutions thereof.

Furthermore, the FGF21 analogs may include the deletion of the 1^{st} amino acid (*i.e.,* histidine) and the 2^{nd} amino acid (*i.e.,* proline) of the native FGF21, and additionally include the substitution of the 167^{th} amino acid (*i.e*., serine) or the 168^{th} amino acid (*i.e*., methionine) of the native FGF21 with threonine, alanine, phenylalanine, or isoleucine. Specifically, the FGF21 analogs may include the deletion of the 1^{st} amino acid (*i.e*., histidine) and the 2^{nd} amino acid (*i.e*., proline) of the native FGF21, and additionally include the substitution of the 167^{th} amino acid (*i.e*., serine) with threonine, the substitution of the 168^{th} amino acid (*i.e*., methionine) with alanine, phenylalanine, or isoleucine, or a combination of at least one of the substitutions thereof.

Additionally, the FGF21 analogs may be an analog, in which one or more amino acids selected from the group consisting of amino acids at positions 5, 6, 7, 8, 9, 168, 172, 176, 177, and 178 of the native FGF21, is(are) substituted with other amino acid(s). The another amino acid(s) may be alanine, but it is not particularly limited thereto.

More specific examples of the FGF21 analogs may include the FGF21 analogs, which have amino acid sequences selected from the group consisting of SEQ ID NO: 46, 47, 48, 49, 50, 51, 52, 53, 54, 55, 56, 57, 58, 59, 60, 61, 62, 63, 64, 65, 66, and 67.

Additionally, the protein conjugate of the present invention may be in a form, in which the protein conjugate is modified with a biodegradable polymer such as polyethylene glycol (PEG) or fatty acid having a binding affinity for *in-vivo* albumin; or a form, in which the protein conjugate is additionally linked to a protein with excellent duration, such as albumin or immunoglobulin; and a form, in which the protein conjugate is enclosed into biodegradable nanoparticles, but is not limited thereto.

In the protein conjugate, the non-peptide polymer may be in a form, in which it is linked at one end to any one selected from a *N*-terminus, a side chain of a lysine residue, and a side chain of an arginine residue of an immunoglobulin Fc; and linked at the other end, by any one selected from a *N*-terminus, a side chain of a lysine residue, a side chain of an arginine residue, a side chain of a histidine residue, and a side chain of a cysteine residue of a physiologically active polypeptide.

As used herein, the term "non-peptide polymer" refers to a biocompatible polymer to which at least two repeating units are linked. The repeating units are linked with each other by any covalent linkage instead of a peptide bond. In the present invention, the terms "non-peptide polymer" and "non-peptide linker" can be used interchangeably.

The non-peptide polymer to be used in the present invention may be selected from the group consisting of polyethylene glycol, polypropylene glycol, an ethylene glycol-propylene glycol copolymer, polyoxyethylated polyol, polyvinyl alcohol, a polysaccharide, dextran, polyvinyl ethyl ether, a biodegradable polymer such as polylactic acid (PLA) and polylactic-glycolic acid (PLGA), lipid polymer, chitin, hyaluronic acid, and a combination thereof, and specifically, polyethylene glycol. Those derivatives which can be easily prepared at the level of the technology in the art also belong to the scope of the present invention.

The peptide linker used in a fusion protein obtained by the existing inframe fusion method has drawbacks in that it is easily cleaved by proteinase *in-vivo,* and thus a sufficient effect of increasing the serum half-life of an active drug by a carrier cannot be obtained as expected. However, in the present invention, the polymer having resistance to proteinase can be used to maintain the serum half-life of the peptide, similar to the carrier. Therefore, any non-peptidyl polymer can be used without limitation in the present invention, as long as it is a polymer having the resistance to proteinase *in-vivo.* The non-peptide polymer has a molecular weight in the range of exceeding 0 kDa to 100 kDa or less, 1 kDa to 100 kDa, and specifically exceeding 0 kDa, for example, in the range of 1 kDa or higher and 20 kDa or less; or in the range of 0.5 kDa to 20 kDa; or 0.5 kDa to 50 kDa; or 5 kDa to 20 kDa; but is not limited thereto. The non-peptide polymer of the present invention, which is linked to the immunoglobulin Fc region, may be one kind of a polymer or a combination of different polymers.

Additionally, the non-peptide polymer may have two or more ends, for example, it may be a non-peptide polymer with two ends or a non-peptide polymer with three ends, but is not limited thereto.

Both ends of the non-peptide polymer can be linked to an amine group or thiol group of an immunoglobulin Fc and one of the two or more polypeptides exhibiting physiological activities.

One or more kinds of non-peptide polymers may be used for one protein conjugate.

For example, the linking may be in a form in which an amine group of a single unit of a polypeptide exhibiting a physiological activity and an amine group of one polypeptide of an immunoglobulin Fc region or a part thereof are linked to both ends of a non-peptide polymer interposed therein, whereas a non-peptide polymer, which connects a single unit of a polypeptide exhibiting a different physiological activity and of another polypeptide of the immunoglobulin Fc region or a part thereof, is linked at one end to an amine group of one of the single unit of the polypeptide exhibiting a different physiological activity, or of the single unit of the polypeptide of the immunoglobulin Fc region or part thereof, and linked at the other end to a thiol group of the other of the single unit of the polypeptide exhibiting a different physiological activity, or of the single unit of the polypeptide of the immunoglobulin Fc region or part thereof.

The non-peptide polymer used in the present invention has reactive groups which can bind to an immunoglobulin Fc region and a physiologically active polypeptide, and thus it can be linked to an immunoglobulin Fc region and a physiologically active polypeptide.

The reactive groups at both ends of the non-peptide polymer may include an aldehyde group, a maleimide group, and a succinimide derivative, but is not limited thereto.

Examples of the aldehyde group may include a propionaldehyde group and a butyraldehyde group, but is not limited thereto.

Examples of the succinimide derivative may include succinimidyl propionate, hydroxy succinimidyl, succinimidyl carboxymethyl, and succinimidyl carbonate.

In the protein conjugate, the covalent linkage may be one formed by reacting a reactive group at one end of a non-peptide polymer selected from the group consisting of an aldehyde group, a maleimide group, and a succinimide derivative with an amino group of the Fc region or the part thereof or an amino group of the physiologically active polypeptide.

When the non-peptidyl polymer has a reactive aldehyde group at both ends, it can minimize non-specific reactions and is effective in binding to a physiologically active polypeptide and an immunoglobulin at both ends, respectively. The final product produced via reductive alkylation by an aldehyde group is more stable than that linked by an amide bond. An aldehyde group selectively reacts to a *N*-terminus at a low pH and it can form a covalent linkage with a lysine residue at a high pH (*e.g.,* pH 9.0).

The reactive groups at both ends of the non-peptide polymer may be the same or different with each other.

For example, the non-peptide polymer may have a maleimide group at one end while having an aldehyde group, *e.g.,* a propionaldehyde group or a butyraldehyde group, at the other end. Additionally, for example, the non-peptide polymer may have an aldehyde group at one end as a reactive group, while having a maleimide group, a succinimide group, an aldehyde group (*e.g.,* a propionaldehyde group or a butyraldehyde group), etc at the other end. For example, when the non-peptide polymer has a reactive aldehyde group at one end and a reactive maleimide group at the other end, non-specific reactions may be minimized and the non-peptide polymer may be effectively linked to a physiologically active polypeptide and an immunoglobulin at its both ends. When polyethylene glycol having a hydroxy group at both ends is used as a non-peptide polymer, the long-acting protein conjugate of the present invention can be prepared by activating the hydroxy group with the various reactive groups described above using a known chemical reaction, or using polyethylene glycol with a modified reactive group available on the commercial market.

As used herein, the term "immunoglobulin Fc region" refers to the heavy chain constant region 2 (CH2) and the heavy chain constant region 3 (CH3) of an immunoglobulin, excluding variable regions of the heavy and light chains. The immunoglobulin Fc region may be one constitution that establishes a moiety of the protein conjugate of the present invention.

The heavy chain constant region of the immunoglobulin Fc region may include a hinge region, but is not limited thereto. Additionally, the immunoglobulin Fc region of the present invention may be an extended Fc region, including a part or the entirety of heavy chain constant region 1 (CH1) and/or the light chain constant region 1 (CL1), but excluding only variable regions of the heavy and light chains of an immunoglobulin, as long as it has an effect substantially the same as or improved compared to its native type. Additionally, the immunoglobulin Fc region of the present invention may be a region in which a considerably long amino acid sequence corresponding to CH2 and/or CH3 is deleted.

For example, the immunoglobulin Fc region of the present invention may include 1) a CH1 domain, a CH2 domain, a CH3 domain, and a CH4 domain; 2) a CH1 domain and a CH2 domain; 3) a CH1 domain and a CH3 domain; 4) a CH2 domain and a CH3 domain; 5) a combination of one or two or more domains among the CH1, CH2, CH3, and CH4 domains and a hinge region of an immunoglobulin (or a part of the hinge region); and 6) a dimer of each domain of the heavy chain constant region and the light chain constant region, but is not limited thereto.

Additionally, in a specific embodiment, the immunoglobulin Fc region may be in a dimeric form.

Additionally, the immunoglobulin Fc region not only includes a native amino acid sequence but also a sequence derivative thereof. An amino acid sequence derivative refers to an amino acid sequence which has a difference in at least one amino acid residue due to deletion, insertion, non-conservative or conservative substitution, or a combination thereof.

For example, the amino acid residues at positions 214 to 238, 297 to 299, 318 to 322, or 327 to 331, which are known to be important in the linking of an immunoglobulin Fc, may be used as suitable sites for modification.

Additionally, other various derivatives are possible, including those which have a deletion of a region capable of forming a disulfide bond, or a deletion of some amino acid residues at the *N*-terminus of native Fc, or an addition of a methionine residue at the *N*-terminus of native Fc. Further, to remove effector functions, a deletion may occur in a complement-binding site, such as a C1q-binding site and an antibody dependent cell mediated cytotoxicity (ADCC) site. Techniques of preparing such sequence derivatives of the immunoglobulin Fc region are disclosed in International Patent Publication Nos. WO 97/34631, WO 96/32478, etc.

Amino acid exchanges in proteins and peptides, which do not generally alter the activity of the proteins or peptides, are known in the art (H. Neurath, R. L. Hill, The Proteins, Academic Press, New York, 1979). The most commonly occurring exchanges are Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, and Asp/Gly. In some cases, the Fc region may be modified by phosphorylation, sulfation, acrylation, glycosylation, methylation, farnesylation, acetylation, amidation, etc.

The above-described Fc derivatives show a biological activity corresponding to that of the Fc region of the present invention or they may be those having improved structural stability of the Fc region against heat, pH, etc.

Further, the immunoglobulin Fc region may be obtained from native forms isolated *in-vivo* from humans or animals such as cows, goats, pigs, mice, rabbits, hamsters, rats, guinea pigs, etc., or may be recombinants or derivatives thereof, obtained from transformed animal cells or microorganisms. In particular, the Fc region may be obtained from a native immunoglobulin by isolating a whole immunoglobulin from a living human or animal body and treating the isolated immunoglobulin with protease. When the whole immunoglobulin is treated with papain, it is cleaved into Fab and Fc regions, whereas when the whole immunoglobulin is treated with pepsin, it is cleaved into pF'c and F(ab)2 fragments. Fc or pF'c may be isolated using size exclusion chromatography, etc. In a more specific embodiment, a human-derived Fc region is a recombinant immunoglobulin Fc region obtained from a microorganism.

Additionally, the immunoglobulin Fc region may be in the form of natural glycans, increased or decreased glycans compared to its native type, or in a deglycosylated form. The increase, decrease, or removal of the immunoglobulin Fc glycans may be achieved by conventional methods such as a chemical method, an enzymatic method, and a genetic engineering method using a microorganism. The immunoglobulin Fc region obtained by removal of glycans from the Fc region shows a significant decrease in binding affinity to the C1q part and a decrease or loss in antibody-dependent cytotoxicity or complement-dependent cytotoxicity, and thus it does not induce unnecessary immune responses *in-vivo.* In this regard, an immunoglobulin Fc region in a deglycosylated or aglycosylated immunoglobulin Fc region may be a more suitable form to meet the original object of the present invention as a drug carrier.

As used herein, the term "deglycosylation" refers to enzymatically removal of sugar moieties from an Fc region, and the term "aglycosylation" refers to an unglycosylated state of the Fc region produced in prokaryotes, more specifically, *E. coli.*

Meanwhile, the immunoglobulin Fc region may be derived from humans or other animals including cows, goats, pigs, mice, rabbits, hamsters, rats, and guinea pigs. In a more specific embodiment, it is derived from humans.

Additionally, the immunoglobulin Fc region may be derived from IgG, IgA, IgD, IgE, IgM, or a combination or hybrid thereof. In a more specific embodiment, it is derived from IgG or IgM, which are among the most abundant proteins in human blood, and in an even more specific embodiment, it is derived from IgG, which is known to enhance the half-lives of ligand-binding proteins. In a yet even more specific embodiment, the immunoglobulin Fc region is an IgG4 Fc region, and in the most specific embodiment, the IgG4 Fc region is an aglycosylated Fc region derived from human IgG4, but is not limited thereto.

In particular, as used herein, the term "combination" means that polypeptides encoding single-chain immunoglobulin Fc regions of the same origin are linked to a single-chain polypeptide of a different origin to form a dimer or multimer. That is, a dimer or multimer may be prepared from two or more fragments selected from the group consisting of IgG Fc, IgA Fc, IgM Fc, IgD Fc, and IgE Fc fragments.

To achieve the present invention, in another aspect, there is provided a method for preparing the conjugate.

The conjugate is the same as explained above.

Specifically, the method includes:
providing (a) a non-peptide polymer, which is linked at one end to a physiologically active polypeptide and comprises a remaining reactive group at the other end, and (b) a physiologically active polypeptide conjugate, in which one or more physiologically active polypeptides are respectively linked to a unit of the immunoglobulin Fc region or a part thereof having the FcRn-binding affinity, wherein the unit of the immunoglobulin Fc region or a part thereof having the FcRn-binding affinity includes a remaining reactive group; and
covalently linking the remaining reactive group of the non-peptide polymer, which is linked to the physiologically active polypeptide of (a), and the remaining reactive group of the immunoglobulin Fc region or a part thereof of the physiologically active polypeptide conjugate of (b).

In particular, the non-peptide polymer of (a) may have the remaining reactive group selected from the group consisting of an aldehyde group, a maleimide group, and a succinimide derivative.

Additionally, the remaining reactive group of an immunoglobulin Fc region or a part thereof of (b) may be positioned in any of the *N*-terminus, a side chain of a lysine residue, and a side chain of an arginine residue of the immunoglobulin Fc region or a part thereof.

Additionally, the physiologically active polypeptide of (a) and the physiologically active polypeptide of (b) may be of the same or different kind with each other, and more specifically, they are of different kind.

Additionally, the physiologically active polypeptide of (a) and the physiologically active polypeptide of (b) may be a single unit, but they are not limited thereto.

To achieve the present invention, in a further aspect, there is provided a composition containing the protein conjugate.

The protein conjugate is the same as explained above.

Additionally, the composition may be one for improving the *in-vivo* duration and stability of a physiologically active polypeptide.

Additionally, the composition may be a pharmaceutical composition. The purpose of the pharmaceutical composition may vary depending on the kind of the physiologically active polypeptide to be contained therein, and diseases such as diabetes and obesity may be included, and the composition may be used as a pharmaceutical composition for the prevention and treatment of these diseases.

The pharmaceutical composition of the present invention may include a pharmaceutically acceptable carrier, excipient, or diluents. The pharmaceutically acceptable carrier, excipient, or diluents may be those which are non-naturally occurring, but are not limited thereto. As used herein, the term "pharmaceutically acceptable" refers to the properties of having a sufficient amount to exhibit a therapeutic effect and not causing adverse effects, and may be easily determined by a skilled person in the art based on the factors well known in the medical field, such as the kind of disease, age, body weight, health status, sex, drug sensitivity of a patient, administration route, administration method, administration frequency, duration of treatment, a drug to be mixed or administered simultaneously in combination, etc.

The pharmaceutically acceptable carrier may include, for oral administration, a binder, a glidant, a disintegrant, an excipient, a solubilizing agent, a dispersant, a stabilizing agent, a suspending agent, a coloring agent, a flavoring agent, etc.; for injections, a buffering agent, a preserving agent, an analgesic, a solubilizing agent, an isotonic agent, a stabilizing agent, etc., which may be combined for use; and for topical administrations, a base, an excipient, a lubricant, a preserving agent, etc., but is not limited thereto.

Meanwhile, examples of carriers, excipients, and diluents suitable for formulation may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oil, etc. Additionally, the composition may further contain a filler, an anti-coagulant, a lubricant, a humectant, a flavoring agent, a preservative, etc.

Additionally, the pharmaceutical composition of the present invention may be prepared in any formulation type selected from the group consisting of tablets, pills, powders, granules, capsules, suspensions, liquid medicine for internal use, emulsions, syrups, sterile injection solutions, non-aqueous solvents, lyophilized formulations, and suppositories.

The composition of the present invention may be prepared in various formulation types by combining with a pharmaceutically acceptable carrier as described above. For example, for oral administration, the composition may be formulated into tablets, troches, capsules, elixirs, suspensions, syrups, wafers, etc. For injections, the composition may be formulated into single-dose ampoules or multidose containers. The composition may be also formulated into solutions, suspensions, tablets, capsules, and sustained-release formulations.

Additionally, the composition may be formulated into a single dosage form suitable for the patient's body, and in a more specific embodiment, it may be formulated into a preparation useful for peptide drugs according to the typical method used in the art to be administered by an oral or parenteral route, such as through skin, intravenously, intramuscularly, intra-arterially, intramedullarily, intrathecally, intraventricularly, pulmonarily, transdermally, subcutaneously, intraperitoneally, intranasally, intragastrically, topically, sublingually, vaginally, or rectally, but is not limited thereto.

Additionally, the peptide may be used by blending with various pharmaceutically acceptable carriers such as physiological saline or organic solvents. In order to increase the stability or absorptivity, carbohydrates such as glucose, sucrose, or dextrans; antioxidants such as ascorbic acid or glutathione; chelating agents; low molecular weight proteins; or other stabilizers may be used.

The administration dose and frequency of the pharmaceutical composition of the present invention are determined by the type of active ingredient(s), along with various factors, such as the disease to be treated, administration route, patient's age, sex, and body weight, and severity of the disease.

The total effective dose of the composition of the present invention may be administered to a patient in a single dose, or may be administered for a long period of time in multiple doses according to a fractionated treatment protocol. In the pharmaceutical composition of the present invention, the content of active ingredient(s) may vary depending on the disease severity. Specifically, the preferable total daily dose of the peptide of the present invention may be approximately 0.0001 µg to 500 mg per 1 kg of body weight of a patient. However, the effective dose of the peptide is determined considering various factors including patient's age, body weight, health conditions, sex, disease severity, diet, and excretion rate, in addition to administration route and treatment frequency of the pharmaceutical composition. In this regard, those skilled in the art may easily determine the effective dose suitable for the particular use of the pharmaceutical composition of the present invention. The pharmaceutical composition according to the present invention is not particularly limited to the formulation and administration route and method, as long as it shows the effects of the present invention.

To achieve the present invention, in another aspect, there is provided a method for treating a target disease including administering the protein conjugate or a composition containing the protein conjugate to a subject in need thereof.

The protein conjugate and a composition containing the protein conjugate are the same as explained above.

The target disease may be appropriately selected according to the type of physiologically active polypeptides applied into the protein conjugate.

Hereinafter, the present invention will be described in more detail with reference to the following examples. However, the examples are provided for illustrative purposes only and the scope of the present invention should not be limited thereto in any manner.

### Example 1. Preparation of a heterodimer conjugate including an insulin analog, imidazo-acetyl exendin-4, and an immunoglobulin Fc

For the pegylation of the *N*-terminus of an insulin analog with an increased half-life (disclosed in WO 2014/133324) compared to that of native insulin using 3.4K PropionALD (2) PEG (a PEG having two propionaldehyde groups; 3 kDa, NOF, JAPAN), the insulin analog and the 3.4K PropionALD (2) PEG were reacted at a molar ratio of 1 : 4, at a protein concentration of 5 mg/mL at a temperature of 4°C to 8°C for about 2 hours. In particular, the reaction was conducted in an environment in which 3 mM sodium cyanoborohydride (NaCNBH₃), a reducing agent, was added to a mixed solvent of 50 mM sodium citrate buffer (pH 5.0) and isopropanol. Upon completion of the reaction, the reactants were applied to the SP Sepharose High Performance (GE, USA) to purify the mono-pegylated insulin analog, using the concentration gradient of 0.25 M potassium chloride and a buffer containing 20 mM sodium citrate (pH 3.0) and ethanol.

Then, the purified mono-pegylated insulin analog and a long-acting imidazo-acetyl exendin-4 (also called "CA-exendin-4") conjugate (disclosed in WO 2008/082274) were reacted. The long-acting imidazo-acetyl exendin-4 conjugate corresponds to a monomer conjugate, which is in a form where a single unit of imidazo-acetyl exendin-4 is linked to a single unit of an immunoglobulin Fc by PEG.

Specifically, the mono-pegylated insulin analog and the long-acting imidazo-acetyl exendin-4 conjugate were reacted at a molar ratio of 1 : 0.2, at a protein concentration of 5 mg/mL to 10 mg/mL at 25°C for about 16 hours. The reaction was conducted in an environment in which 20 mM sodium cyanoborohydride, a reducing agent, was added to 100 mM HEPES buffer (pH 8.2) and sodium chloride. Upon completion of the reaction, the reactants were applied to the PolyWAX LP (PolyLC, USA) using the concentration gradient of 20 mM Tris (pH 7.0) and 0.5 M sodium chloride, and applied to Source15Q (GE, USA) using concentration gradient of 0.25 M sodium chloride and 20 mM bis-Tris (pH 6.0), in order to purify a conjugate, in which an insulin analog and CA-exendin-4 are covalently linked to a unit of an immunoglobulin Fc by PEG, respectively.

### Example 2. Preparation of a homodimer conjugate including two units of insulin analog and an immunoglobulin Fc

For the pegylation of the *N*-terminus of an insulin analog with an increased half-life (disclosed in WO 2014/133324) compared to that of native insulin using 3.4K PropionALD (2) PEG (a PEG having two propionaldehyde groups; 3 kDa, NOF, JAPAN), the insulin analog and the 3.4K PropionALD (2) PEG were reacted at a molar ratio of 1 : 4, at a protein concentration of 5 mg/mL at a temperature of 4°C to 8°C for about 2 hours. In particular, the reaction was conducted in an environment in which 3 mM sodium cyanoborohydride, a reducing agent, was added to a mixed solvent of 50 mM sodium citrate buffer (pH 5.0) and isopropanol. Upon completion of the reaction, the reactants were applied to the SP Sepharose High Performance (GE, USA) to purify the mono-pegylated insulin analog, using the concentration gradient of 0.25 M potassium chloride and a buffer containing 20 mM sodium citrate (pH 3.0) and 45% ethanol.

Then, the purified mono-pegylated insulin analog and a long-acting insulin conjugate (disclosed in WO 2014/133324) in the form of a monomer conjugate were reacted. The long-acting insulin conjugate corresponds to a monomer conjugate, which is in a form where a single unit of insulin analog is linked to a single unit of an immunoglobulin Fc by PEG. The insulin analog of the corresponding monomer conjugate is the same kind as the mono-pegylated insulin analog.

Specifically, the mono-pegylated insulin analog and the long-acting insulin conjugate in the form of a monomer conjugate were reacted at a molar ratio of 1 : 0.2, at a protein concentration of 15 mg/mL at 25°C for about 16 hours. The reaction was conducted in an environment in which 20 mM sodium cyanoborohydride, a reducing agent, was added to 100 mM HEPES buffer (pH 8.2) and sodium chloride. Upon completion of the reaction, the reactants were applied to the Sephadex G25 (GE, USA) and the buffer was replaced with 20 mM bis-Tris (pH 6.0). Then, the reactants were applied to the Source 15Q (GE, USA) using the concentration gradient of 0.25 M sodium chloride and 20 mM bis-Tris (pH 6.0), and applied to the Source ISO (GE, USA) using the concentration gradient of 1.3 M ammonium sulfate and 20 mM Tris (pH 7.5), to purify the conjugate, in which two units of an insulin analog is covalently linked to a single unit of an immunoglobulin Fc by PEG, respectively.

### Example 3. Preparation of a homodimer conjugate including two units of imidazo-acetyl exendin-4 and an immunoglobulin Fc

For the pegylation of the lysine of imidazo-acetyl exendin-4 (Bachem, USA) (disclosed in WO 2008/082274) using 3.4K PropionALD (2) PEG (a PEG having two propionaldehyde groups; 3.4 kDa, NOF, JAPAN), the imidazo-acetyl exendin-4 and the 3.4K PropionALD (2) PEG were reacted at a molar ratio of 1 : 15, at a protein concentration of 6 mg/mL at a temperature of 4°C to 8°C for about 13.5 hours. In particular, the reaction was conducted in an environment in which 20 mM sodium cyanoborohydride, a reducing agent, was added to a mixed solvent of 100 mM HEPES buffer (pH 7.5) and isopropanol. Upon completion of the reaction, the reactants were applied to the Source 15S (GE, USA) using the concentration gradient of 0.25 M potassium chloride and a buffer containing 20 mM sodium citrate (pH 3.0) and isopropanol, in order to purify the mono-pegylated imidazo-acetyl exendin-4.

Then, the purified mono-pegylated imidazo-acetyl exendin-4 and imidazo-acetyl exendin-4 conjugate (disclosed in WO 2008/082274) in the form of a monomer conjugate were reacted at a molar ratio of 1 : 5, at a protein concentration of 20 mg/mL at a temperature of 4°C to 8°C for about 20 hours. The reaction was conducted in an environment in which 20 mM sodium cyanoborohydride, a reducing agent, was added to 100 mM potassium phosphate buffer (pH 6.0). Upon completion of the reaction, the unreacted reactants were removed by applying to the Source Phenyl (GE, USA) using the concentration gradient of sodium chloride and 5 mM Tris (pH 7.0), and then reactants were applied to the Source15Q (GE, USA) using the concentration gradient of 20 mM Tris (pH 7.5) and sodium chloride, and applied to SourceISO (GE, USA) using the concentration gradient of ammonium sulfate and 20mM Tris (pH 7.5) in order to purify the homodimer conjugate, in which two units of imidazo-acetyl exendin-4 are covalently linked to a single unit of an immunoglobulin Fc by PEG, respectively.

### Example 4. Preparation of a heterodimer conjugate including an insulin analog, a glucagon analog, and an immunoglobulin Fc

For the pegylation of the cysteine residue of a glucagon analog using maleimide-lOK PEG-PropionALD (a PEG having a maleimide group and a propionaldehyde group, respectively; 10 kDa, NOF, JAPAN), the glucagon analog and the maleimide-10K PEG-PropionALD were reacted at a molar ratio of 1 : 1.3, at a protein concentration of 3 mg/mL at a temperature of 4°C to 8°C for about 1 hour. Here, the glucagon analog is an analog with a modification on the sequence of native glucagon and it includes a cysteine residue. In particular, the reaction was conducted in an environment of a mixed solvent of 50 mM Tris buffer (pH 7.5) and isopropanol. Upon completion of the reaction, the reactants were applied to the SP Sepharose High Performance (GE, USA) to purify the mono-pegylated glucagon analog using the concentration gradient of a buffer containing 20 mM sodium citrate (pH 3.0) and ethanol and 0.25 M potassium chloride.

Then, the purified mono-pegylated glucagon analog and a long-acting insulin analog conjugate (disclosed in WO 2014/133324) in the form of a monomer conjugate were reacted at a molar ratio of 1 : 7.5, at a protein concentration of 25 mg/mL at a temperature of 4°C for about 16 hours. The reaction was conducted in an environment in which 20 mM sodium cyanoborohydride, a reducing agent, was added to a mixed solvent of a 100 mM potassium phosphate buffer (pH 6.0) and isopropanol. Upon completion of the reaction, the reactants were applied to the Source 15 Q (GE, USA) using the concentration gradient of 20 mM bis-Tris (pH 6.5) and 0.25 M sodium chloride, and applied to SourceISO (GE, USA) along the concentration gradient of ammonium sulfate and 20 mM sodium citrate (pH 5.2), in order to purify the heterodimer conjugate, in which an insulin analog and a glucagon analog are covalently linked to a single unit of an immunoglobulin Fc by PEG, respectively.

### Example 5. Preparation of a heterodimer conjugate including imidazo-acetyl exendin-4 (CA-exendin-4), a glucagon analog, and an immunoglobulin Fc

For the pegylation of the cysteine residue of a glucagon analog using maleimide-lOK PEG-PropionALD (a PEG having a maleimide group and a propionaldehyde group, respectively; 10 kDa, NOF, JAPAN), the mono-pegylated glucagon analog was purified under the reaction condition for pegylation and the purification condition as in Example 4. Here, the glucagon analog is an analog with a modification on the sequence of native glucagon and it includes a cysteine residue.

Then, the purified mono-pegylated glucagon analog and a long-acting imidazo-acetyl exendin-4 conjugate (disclosed in WO 2008/082274) in the form of a monomer conjugate were subjected to the reaction condition for conjugation and the purification condition as in Example 4 to purify the heterodimer conjugate, in which the imidazo-acetyl exendin-4 and the glucagon analog are covalently linked to a single unit of an immunoglobulin Fc by PEG, respectively.

### Example 6. Preparation of a heterodimer conjugate including imidazo-acetyl exendin-4 (CA-exendin-4), fibroblast growth factor 21 (FGF21), and an immunoglobulin Fc

For the pegylation of the *N*-terminus of fibroblast growth factor 21 using 3.4K PropionALD (2) PEG (a PEG having two propionaldehyde groups; 3.4 kDa, NOF, JAPAN), the fibroblast growth factor 21 and the 3.4K PropionALD (2) PEG were reacted at a molar ratio of 1 : 3, at a protein concentration of 5 mg/mL at a temperature of 4°C to 8°C for about 2 hours. In particular, the fibroblast growth factor 21 was obtained by transforming *E. coli* with an FGF21 expression vector prepared based on the FGF21 cDNA (OriGene, RC204538), expressing FGF21 with the periplasm of *E. coli,* followed by extraction and purification of the resultant. In particular, the reaction was conducted in an environment in which 20 mM sodium cyanoborohydride, a reducing agent, was added to a 100 mM potassium phosphate buffer (pH 6.0). Upon completion of the reaction, the reactants were applied to the Source15Q (GE, USA) to purify the mono-pegylated fibroblast growth factor 21 using the concentration gradient of 0.25 M NaCl and a buffer containing 20 mM Tris (pH 7.0).

Then, the purified mono-pegylated fibroblast growth factor 21 and a long-acting imidazo-acetyl exendin-4 conjugate (disclosed in WO 2008/082274) were reacted at a molar ratio of 1 : 3, at a protein concentration of 30 mg/mL at a temperature of 4°C to 8°C for about 16 hours. The reaction was conducted in an environment in which 20 mM sodium cyanoborohydride, a reducing agent, was added to a 100 mM potassium phosphate buffer (pH 6.0). Upon completion of the reaction, the reactants were applied to the Source15Q (GE, USA) using the concentration gradient of 20 mM bis-Tris (pH 6.0) and 0.25 M sodium chloride, and applied to the Source ISO (GE, USA) using the concentration gradient of ammonium sulfate and 20 mM Tris (pH 7.5), in order to purify the heterodimer conjugate, in which imidazo-acetyl exendin-4 and fibroblast growth factor 21 are covalently linked to a single unit of an immunoglobulin Fc by PEG, respectively.

### Example 7. Analysis of a heterodimer conjugate including an insulin analog, imidazo-acetyl exendin-4 (CA-exendin-4), and an immunoglobulin Fc

To confirm the purity of the heterodimer conjugate prepared according to Example 1, RP-HPLC, IE-HPLC, and SE-HPLC analyses were conducted. In Table 2, RP-HPLC (%), IE-HPLC (%), and SE-HPLC (%) represent the purity of the heterodimer conjugate in terms of area (%).

**[Table 2]**

| Material Name | RP-HPLC (%) | IE-HPLC (%) | SE-HPLC (%) |
|---|---|---|---|
| Heterodimer Conjugate | 97.4 | 95.3 | 99.4 |

As can be seen in the above results, the results of the purity analyzed by RP-HPLC, IE-HPLC, and SE-HPLC were all confirmed to be 95% or higher.

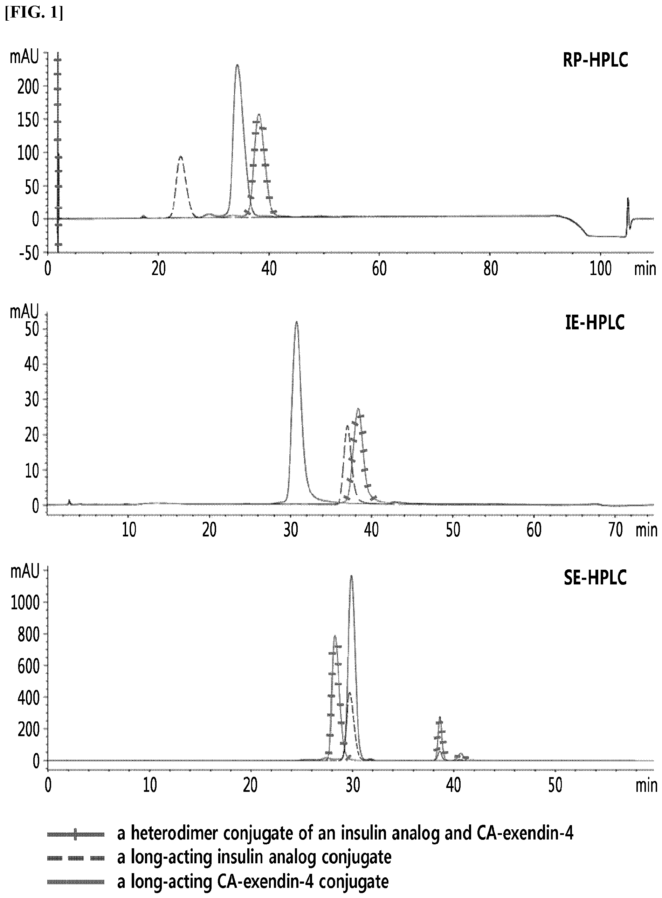

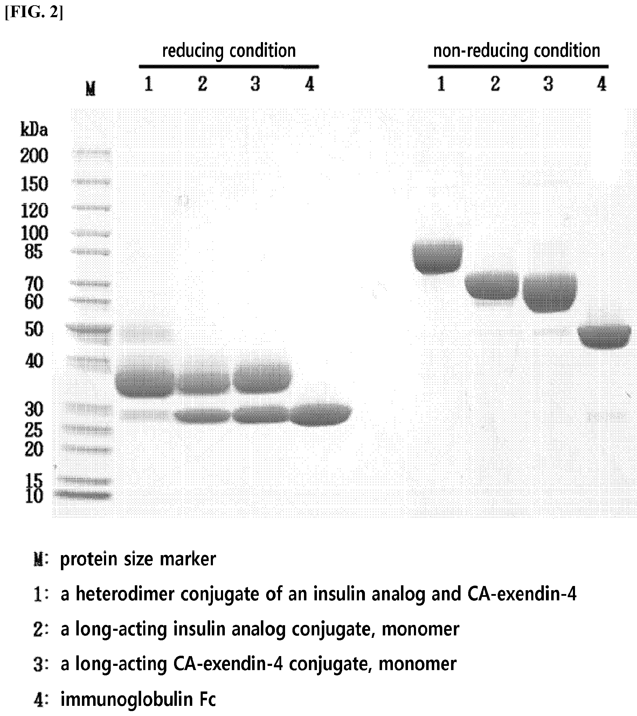

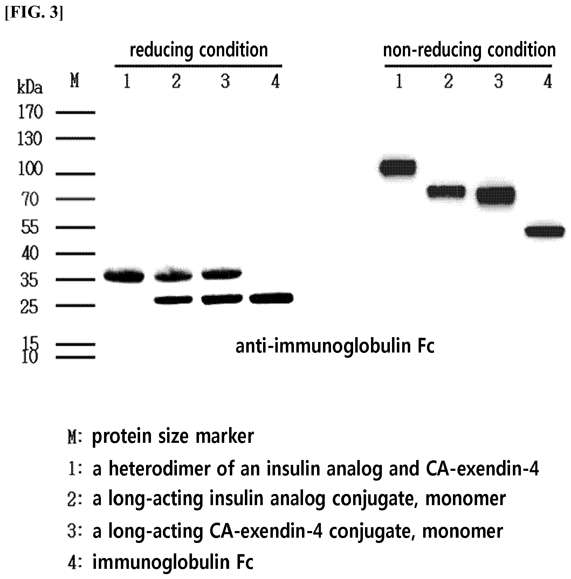

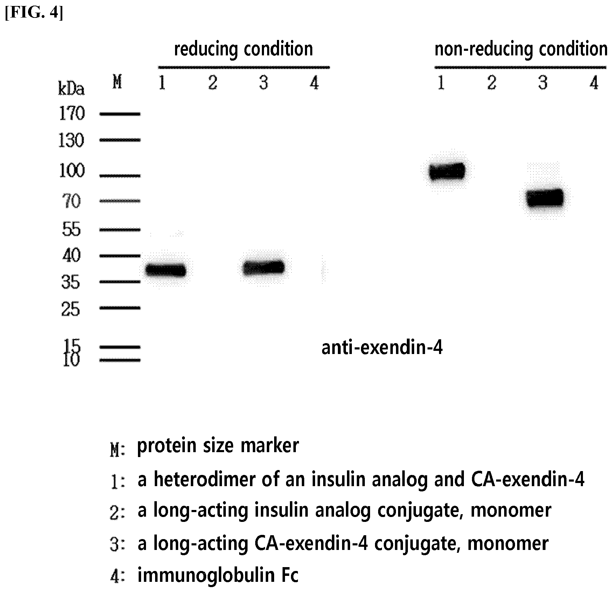

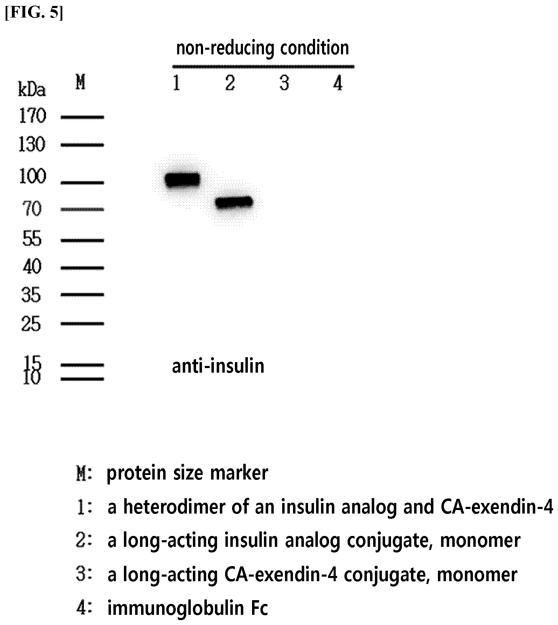

The heterodimer conjugate was compared by analyzing along with the monomer conjugates, *i.e.,* a long-acting insulin analog conjugate and a long-acting imidazo-acetyl exendin-4 conjugate. The respective result of RP-HPLC, IE-HPLC, and SE-HPLC is shown in FIG. 1 and the SDS-PAGE result is shown in FIG. 2. The western blot results using anti-immunoglobulin Fc, anti-exendin-4, and anti-insulin are shown in FIG. 3, FIG. 4, and FIG. 5, respectively.

### Example 8. Analysis of a homodimer conjugate including two units of insulin analog and an immunoglobulin Fc

To confirm the purity of the long-acting insulin analog homodimer conjugate prepared according to Example 2, IE-HPLC and SE-HPLC analyses were conducted. In Table 3, IE-HPLC (%) and SE-HPLC (%) represent the purity of t the long-acting insulin analog homodimer conjugate in terms of area (%).

**[Table 3]**

| Material Name | IE-HPLC (%) | SE-HPLC (%) |
|---|---|---|
| a long-acting insulin analog homodimer conjugate | 99.3 | 99.2 |

As can be seen in the above results, the results of the purity analyzed by IE-HPLC and SE-HPLC were all confirmed to be 95% or higher.

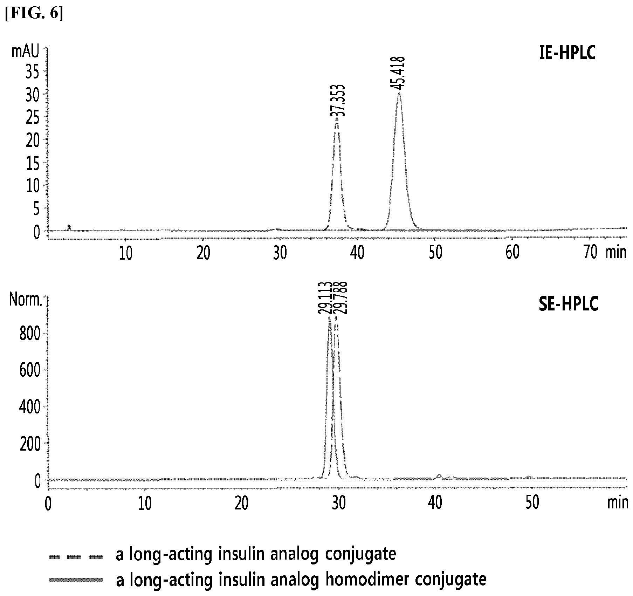

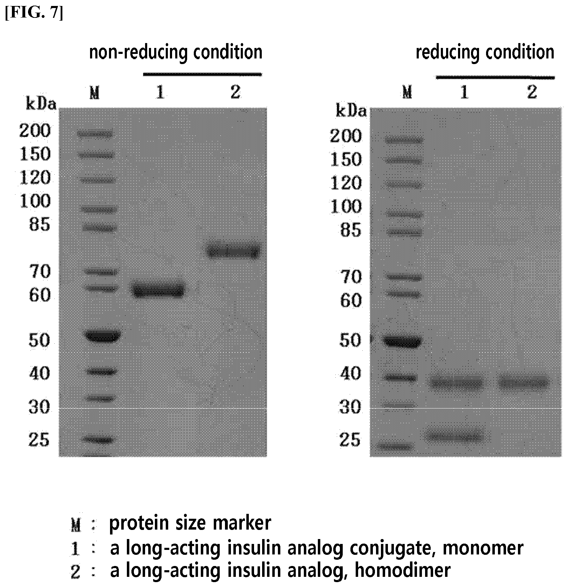

The long-acting insulin analog homodimer conjugate was compared by analyzing along with the long-acting insulin analog monomer conjugate. The respective result of IE-HPLC and SE-HPLC is shown in FIG. 6 and the SDS-PAGE result is shown in FIG. 7.

### Example 9. Analysis of a homodimer conjugate including two units of imidazo-acetyl exendin-4 (CA-exendin-4) and an immunoglobulin Fc

To confirm the purity of the the long-acting imidazo-acetyl exendin-4 homodimer conjugate prepared according to Example 3, SE-HPLC and RP-HPLC analyses were conducted. In Table 4, SE-HPLC (%) and RP-HPLC (%) represent the purity of the the long-acting imidazo-acetyl exendin-4 homodimer conjugate in terms of area (%).

**[Table 4]**

| Material Name | SE-HPLC (%) | RP-HPLC (%) |
|---|---|---|
| a long-acting imidazo-acetyl exendin-4 homodimer conjugate | 98.2 | 81.2 |

As can be seen in the above results, the results of the purity analyzed by IE-HPLC and SE-HPLC were all confirmed to be 80% or higher.

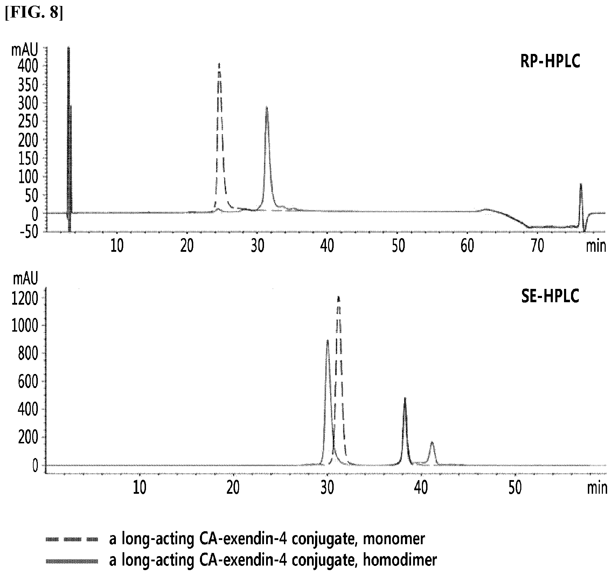

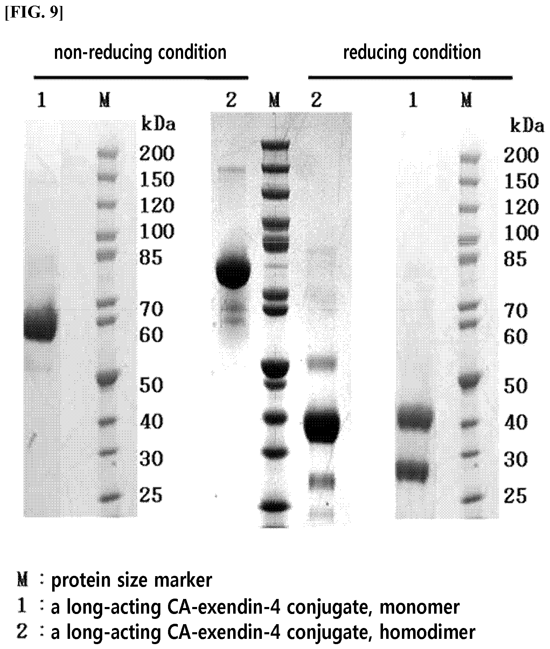

The long-acting imidazo-acetyl exendin-4 homodimer conjugate was compared by analyzing along with the long-acting imidazo-acetyl exendin-4 monomer conjugate. In particular, the respective result of SE-HPLC and RPE-HPLC is shown in FIG. 8 and the SDS-PAGE result is shown in FIG. 9.

### Example 10. Analysis of a heterodimer conjugate including an insulin analog, a glucagon analog, and an immunoglobulin Fc

To confirm the purity of the heterodimer conjugate prepared according to Example 4, RP-HPLC, IE-HPLC, and SE-HPLC analyses were conducted. In Table 5, RP-HPLC (%), IE-HPLC (%), and SE-HPLC (%) represent the purity of the heterodimer conjugate in terms of area (%).

**[Table 5]**

| Material Name | RP-HPLC (%) | IE-HPLC (%) | SE-HPLC (%) |
|---|---|---|---|
| Heterodimer Conjugate | 96.7 | 99.0 | 95.6 |

As can be seen in the above results, the results of the purity analyzed by RP-HPLC, IE-HPLC, and SE-HPLC were all confirmed to be 95% or higher.

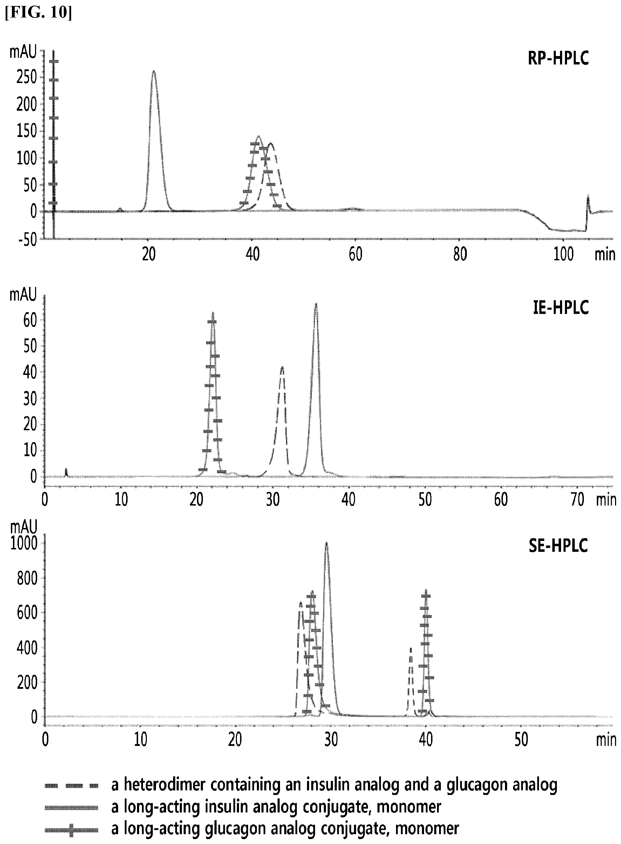

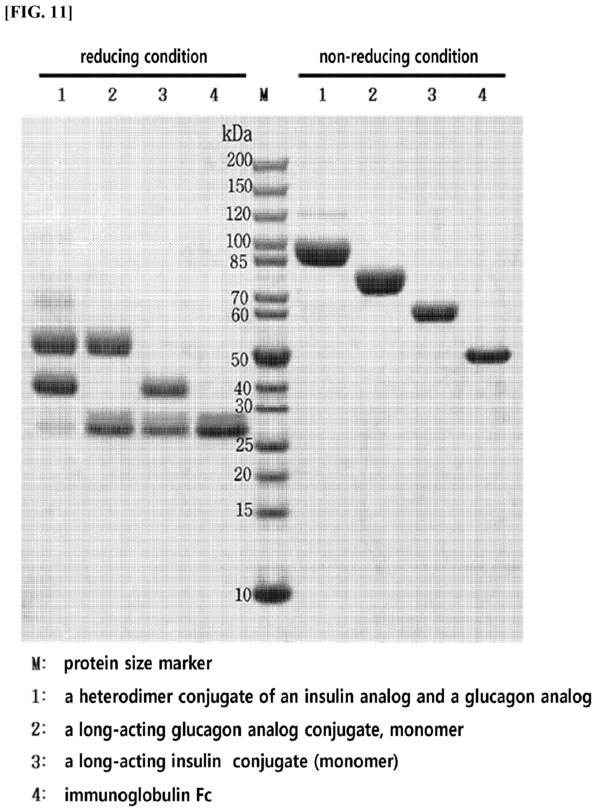

The heterodimer conjugate was compared by analyzing along with the long-acting insulin analog conjugate and the long-acting glucagon analog conjugate. In particular, the respective result of RP-HPLC, IE-HPLC, and SE-HPLC is shown in FIG. 10 and the SDS-PAGE result is shown in FIG. 11.

### Example 11. Analysis of a heterodimer conjugate including imidazo-acetyl exendin-4 (CA-exendin-4), a glucagon analog, and an immunoglobulin Fc

To confirm the purity of the heterodimer conjugate prepared according to Example 5, RP-HPLC, IE-HPLC, and SE-HPLC analyses were conducted. In Table 6, RP-HPLC (%), IE-HPLC (%), and SE-HPLC (%) represent the purity of the heterodimer conjugate in terms of area (%).

**[Table 6]**

| Material Name | RP-HPLC (%) | IE-HPLC (%) | SE-HPLC (%) |
|---|---|---|---|
| Heterodimer Conjugate | 96.7 | 95.9 | 91.5 |

As can be seen in the above results, the results of the purity analyzed by RP-HPLC, IE-HPLC, and SE-HPLC were all confirmed to be 90% or higher.

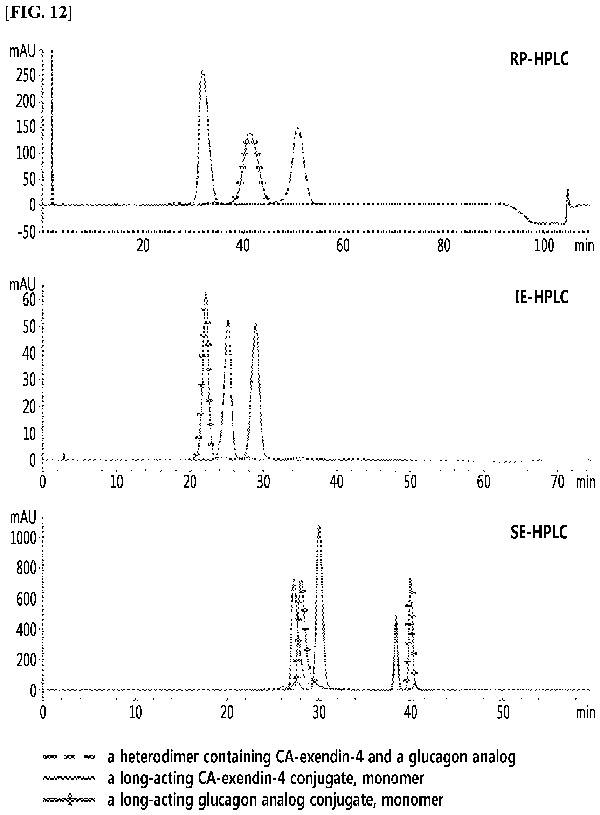

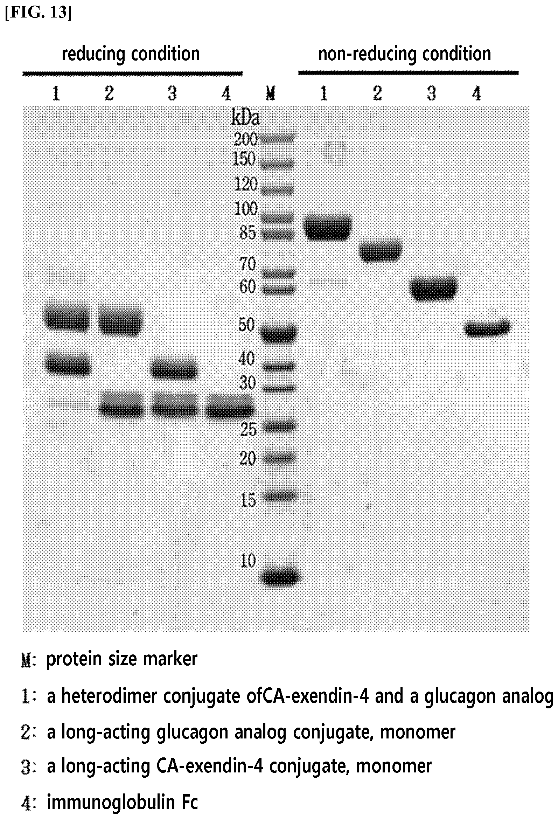

The heterodimer conjugate was compared by analyzing along with the long-acting imidazo-acetyl exendin-4 conjugate and the long-acting glucagon analog conjugate. In particular, the respective result of RP-HPLC, IE-HPLC, and SE-HPLC is shown in FIG. 12 and the SDS-PAGE result is shown in FIG. 13.

### Example 12. Analysis of a heterodimer conjugate including imidazo-acetyl exendin-4 (CA-exendin-4), fibroblast growth factor 21 (FGF21), and an immunoglobulin Fc

To confirm the purity of the heterodimer conjugate prepared according to Example 6, RP-HPLC, IE-HPLC, and SE-HPLC analyses were conducted. In Table 7, RP-HPLC (%), IE-HPLC (%), and SE-HPLC (%) represent the purity of the heterodimer conjugate in terms of area (%).

**[Table 7]**

| Material Name | RP-HPLC (%) | IE-HPLC (%) | SE-HPLC (%) |
|---|---|---|---|
| Heterodimer Conjugate | 97.6 | 97.6 | 97.5 |

As can be seen in the above results, the results of the purity analyzed by RP-HPLC, IE-HPLC, and SE-HPLC were all confirmed to be 95% or higher.

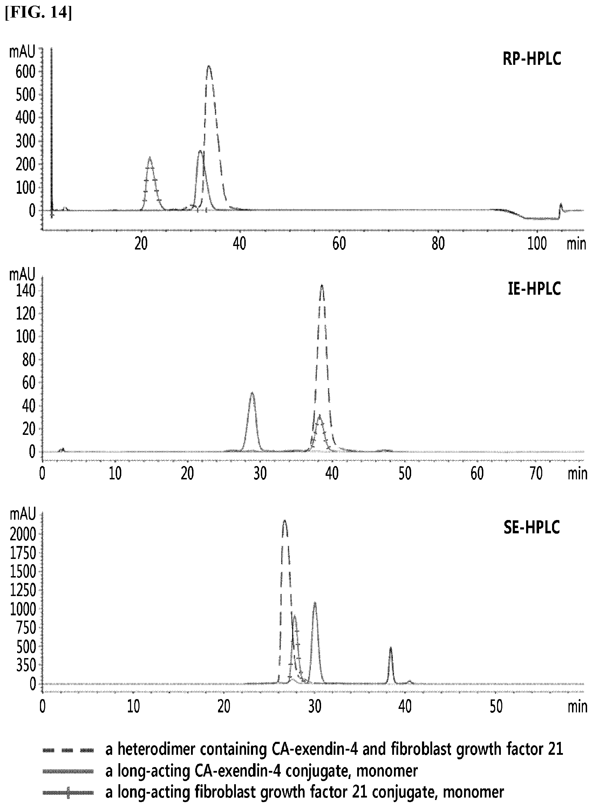

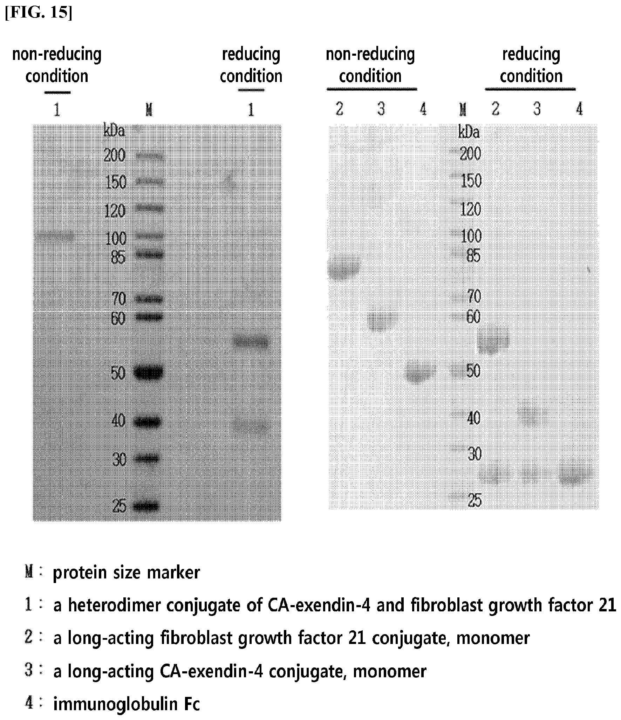

The heterodimer conjugate was compared by analyzing along with the long-acting imidazo-acetyl exendin-4 conjugate and the long-acting fibroblast growth factor 21 conjugate. In particular, the respective result of RP-HPLC, IE-HPLC, and SE-HPLC is shown in FIG. 14 and the SDS-PAGE result is shown in FIG. 15.

### Example 13. Analysis of the titer of a heterodimer conjugate including an insulin analog, imidazo-acetyl exendin-4 (CA-exendin-4), and an immunoglobulin Fc

To confirm the titers of the heterodimer including the insulin analog and exendin-4 prepared in Example 1, the homodimer of the insulin analog prepared in Example 2, and the homodimer of the imidazo-acetyl exendin-4 prepared in Example 3, the method of measuring *in-vitro* cell activity using each of the prepared cell lines was employed.

The respective cell line was transformed to enable the expression of human GLP-1 receptors and human insulin receptor β gene in Chinese hamster ovary (CHO) cells and they are suitable for measuring the activities of GLP-1 and insulin. Accordingly, the activities of each of the homodimers were measured using each of the transformed cell lines.

For the confirmation of the titer of the insulin analog and the comparison of the titer of the heterodimer with that of the long-acting insulin analog monomer conjugate, human insulin were subjected to a 3-fold continuous dilution from 344.4 nM to 0.2 nM, and also the the long-acting insulin analog monomer conjugate, the heterodimer including an insulin analog and imidazo-acetyl exendin-4, and the homodimer of an insulin analog, and an immunoglobulin Fc were subjected to a 3-fold continuous dilution from 20037.1 nM to 9.2 nM. The cultured CHO cells expressing the human insulin receptor β were washed with a KRB buffer, and each of the materials continuously diluted in an amount of 100 µL was added to the CHO cells, and cultured in a CO₂ incubator at 37°C for 10 minutes. Then, cell lysis buffer was added to the cells in an amount of 25 µL for cell lysis, and the cell lysate was applied to a Phospho-Insulin Receptor β Sandwich ELISA kit (Cell signaling, USA) to measure the phosphorylated insulin receptor β, and EC₅₀ values were calculated therefrom and compared with each other. The EC₅₀ values and the relative titers relative to human insulin are shown in Table 8 below.

For the confirmation of the titer of imidazo-acetyl exendin-4 and the comparison with the long-acting imidazo-acetyl exendin-4 monomer conjugate, the imidazo-acetyl exendin-4 were subjected to a 4-fold continuous dilution from 50 nM to 0.000048 nM, and the heterodimer including an insulin analog and imidazo-acetyl exendin-4, the homodimer of imidazo-acetyl exendin-4, and an immunoglobulin Fc were subjected to a 4-fold continuous dilution from 400 nM to 0.00038 nM. The culture medium was removed from the cultured CHO cells expressing the human GLP-1 receptor, and each material, which was continuously diluted, was added to the cells in an amount of 5 µL, and cultured at room temperature for 15 minutes. Then, a detection mix containing cell lysis buffer was added to the cells in an amount of 10 µL for cell lysis, and reacted at room temperature for 90 minutes. The cell lysate, upon completion of the reaction, was applied to the LANCE cAMP kit (PerkinElmer, USA) to measure the amount of accumulated cAMP, and EC₅₀ values were calculated therefrom and compared with each other. The EC₅₀ values and the relative titers relative to imidazo-acetyl exendin-4 are shown in Table 8 below.

As a result of the experiment, it was confirmed that in a case of a conjugate, in which two or more kinds of physiologically active polypeptide materials are linked to an immunoglobulin Fc by a non-peptide polymer, respectively, the conjugate had a physiological activity similar to that of a conjugate, in which a single kind of a physiologically active polypeptide material is linked to an immunoglobulin Fc by a non-peptide polymer.

**[Table 8] Comparison of in-vitro activities of novel insulin analogs and imidazo-acetyl exendin-4 (CA-exendin-4) in GLP-1 receptors and insulin receptors**

| **Material Name** | **IR-β phosphorylation** | | **cAMP assay** | |
|---|---|---|---|---|
| | **EC₅₀ (nmol/L)** | **Relative activity (% *vs.* rh-INS)** | **EC₅₀ (nmol/L)** | **Relative activity (% *vs.* Exd4)** |
| Human Insulin (hr-Insulin) | 6.7 ± 1.1 | **100%** | *n*/*a* | - |
| CA-Exendin-4 | *n*/*a* | - | 0.03 ± 0.02 | **100%** |
| Monomer Conjugate of Novel Insulin Analog | 336.2 ± 23.4 | **2.0 ± 0.4%** | *n*/*a* | - |
| Monomer Conjugate of CA-Exendin-4 | *n*/*a* | - | 0.59 ± 0.37 | **5.5 ± 0.3%** |
| Heterodimer conjugate | 210.9 ± 29.0 | **3.2 ± 0.5%¹⁾** | 0.67 ± 0.13 | **4.9 ± 2.9%¹⁾** |
| Homodimer Conjugate of Novel Insulin Analog | 122.3 ± 16.7 | **5.6 ± 1.2%²⁾** | *n*/*a* | - |
| Homodimer conjugate of CA-Exendin-4 | *n*/*a* | - | 0.23 ± 0.06 | **13.2 ± 4.7%²⁾** |
| Immunoglobulin Fc | >1000 | - | >100 | - |

| | | | | |
|---|---|---|---|---|
| 1) No statistical significance relative to each monomer conjugates. (*p*≥0.05, one-way ANOVA) 2) Presence of statistical significance relative to each monomer conjugates. (*p*<0.05, one-way ANOVA) | | | | |

### Example 14. In-vitro titer analysis of a heterodimer conjugate including an insulin analog, a glucagon analog, and an immunoglobulin Fc

For the confirmation of the titer of a heterodimer including the insulin analog and the glucagon analog prepared in Example 4, the method of measuring *in-vitro* cellular activity was employed.

First, for analyzing the titer of insulin, a glucose uptake (or lipid synthesis ability) test was conducted using the mouse-derived 3T3-L1 cell line differentiated into adipocytes. The 3T3-L1 cell line (ATCC, CL-173) was maintained by subculturing two or three times per week using Dulbeco's Modified Eagle's Medium (DMEM, Gibco, Cat. No. 12430) containing 10% Newborn Calf Serum (NBCS). The 3T3-L1 cell line was suspended in the medium for differentiation (DMEM containing 10% FBS), inoculated into a 48-well plate to a concentration of 5 × 10⁴ cells/well, and cultured at 37°C for 48 hours. For the differentiation into adipocytes, 1 µg/mL of human insulin (Sigma, Cat. No. 19278), 0.5 mM of 3-isobutyl-1-methylxanthine (IBMX, Sigma, Cat. No. 15879), and 1 µM dexamethasone (Sigma, Cat. No. D4902) were mixed to the differentiation medium. The mixtures was added to each well in an amount of 250 µL/well after removing the existing medium. 48 hours thereafter, the medium in each well was replaced with the medium containing only human insulin (1 µg/mL). Then, the medium in each well was replaced with the differentiation medium containing the human insulin (1 µg/mL) every 48 hours and the induction of differentiation into adipocytes were confirmed for 10 days to 14 days.

For the glucose uptake test, the cells terminally differentiated into adipocytes were washed once with serum-free DMEM medium and serum-free DMEM medium was added into each well in an amount of 250 µL to induce serum depletion for 4 hours. Human insulin, human glucagon, a long-acting insulin analog monomer, a long-acting glucagon analog monomer, an insulin/glucagon analog heterodimer and an immunoglobulin Fc were prepared by sequentially diluting 10-fold from 10,000 nM to 0.01 nM with serum-free DMEM medium. Each of the thus-prepared samples was added to the cells in an amount of 250 µL, respectively, and cultured in a 5% CO₂ incubator at 37°C for 24 hours. For the measurement of the residual amount of glucose in the medium upon completion of cultivation, 200 µL of the medium was collected, diluted 5-fold with D-PBS, respectively, and subjected to GOPOD analysis (GOPOD Assay Kit, Megazyme, Cat. No. K-GLUC). The residual glucose concentration in the medium was calculated based on the absorbance of the standard glucose solution, and EC₅₀ values for each of the samples with respect to glucose uptake ability was calculated and the result is shown in Table 9 below.

Then, for analyzing the titer of glucagon, the method of measuring *in-vitro* cellular cAMP activity was employed using the transformed cell line to enable the expression of human glucagon receptor in the Chinese hamster ovary (CHO) cells explicitly specified above.

For the confirmation of the titer of a heterodimer including an insulin analog, a glucagon analog, and an immunoglobulin Fc, and the comparison with the long-acting glucagon analog conjugate, human glucagon was subjected to a 4-fold continuous dilution from 50 nM to 0.000048 nM, and a long-acting glucagon analog monomer conjugate; a long-acting insulin analog monomer conjugate; a heterodimer including an insulin analog, a glucagon analog, and an immunoglobulin Fc; and lastly an immunoglobulin Fc were subjected to a 4-fold continuous dilution from 400 nM to 0.00038 nM. The culture medium was removed from the cultured CHO cells expressing the human glucagon receptor, and each material, which was continuously diluted, was added to the cells in an amount of 5 µL, and cultured at room temperature for 15 minutes. Then, a detection mix containing cell lysis buffer was added to the cells in an amount of 10 µL for cell lysis, and reacted at room temperature for 90 minutes. The cell lysate, upon completion of the reaction, was applied to the LANCE cAMP kit (PerkinElmer, USA) to measure the amount of accumulated cAMP, and EC₅₀ values were calculated therefrom and compared with each other. The EC₅₀ values and the relative titers relative to human glucagon are shown in Table 9 below.

As shown in Table 9, the heterodimer including an insulin analog and a glucagon analog according to the present invention showed 10.1% of insulin activity and 10.7% of glucagon activity, and these values were confirmed to be similar to those of the long-acting insulin analog monomer and the long-acting glucagon analog monomer, respectively.

From the above results, it was confirmed that in a case of a conjugate, in which two or more kinds of physiologically active polypeptide materials are linked to an immunoglobulin Fc by a non-peptide polymer, respectively, the conjugate had a physiological activity similar to that of a conjugate, in which a single kind of a physiologically active polypeptide material is linked to an immunoglobulin Fc by a non-peptide polymer.

**[Table 9] Comparison of in-vitro activity of an insulin analog and a glucagon analog**

| **Material Name** | **Glucose uptake (Insulin)** | | **cAMP assay (Glucagon)** | |
|---|---|---|---|---|
| | **EC₅₀ (nM)** | **Relative activity (% vs. Insulin)** | **EC₅₀ (nM)** | **Relative activity (% vs. GCG)** |
| **Insulin** | 9.0 / 11.0 | **100** | ND | ND |
| **Glucagon (GCG)** | ND | ND | 0.011 | **100** |
| **Monomer Conjugate of Insulin Analog** | 130 | **8.5** | ND | ND |
| **Monomer Conjugate of Glucagon Analog** | ND | ND | 0.120 | **9.1** |
| **Heterodimer Conjugate of Insulin/Glucagon Analog** | 109 | **10.1** | 0.101 | **10.7** |
| **Immunoglobulin Fc** | ND | ND | ND | ND |

| | | | | |
|---|---|---|---|---|
| ND : Not determined, because the dose-response curve was not saturated up to highest evaluated concentration. | | | | |

### Example 15. In-vitro titer analysis of a heterodimer conjugate including imidazoacetyl exendin-4 (CA-exendin-4), a glucagon analog, and an immunoglobulin Fc

For the confirmation of the titer of a heterodimer including the imidazo-acetyl exendin-4 and glucagon analog prepared in Example 5, the method of measuring *in-vitro* cAMP activity using each cell line was employed.

The respective cell line was transformed to enable the expression of human glucagon receptors and human glucagon-like peptide-1 (GLP-1) receptor gene in Chinese hamster ovary (CHO) cells and they are suitable for measuring the activities of a glucagon analog and imidazo-acetyl exendin-4. Accordingly, the activities of each of the heterodimers were measured using each of the transformed cell lines.

For the confirmation of the titer of a heterodimer including the imidazo-acetyl exendin-4 and glucagon analog and an immunoglobulin Fc, and the comparison with long-acting imidazo-acetyl exendin-4 monomer conjugate and the long-acting glucagon analog monomer conjugate, human glucagon and glucagon-like peptide-1 (GLP-1) were subjected to a 4-fold continuous dilution from 50 nM to 0.000048 nM, and a long-acting glucagon analog monomer conjugate; a long-acting imidazo-acetyl exendin-4 monomer conjugate; a heterodimer including a glucagon analog, imidazo-acetyl exendin-4, and an immunoglobulin Fc; and lastly an immunoglobulin Fc were subjected to a 4-fold continuous dilution from 400 nM to 0.00038 nM. The culture medium was removed from the cultured CHO cells, in which the human glucagon receptors and human glucagon-like peptide-1 receptors were expressed, each material, which was continuously diluted, was added to the cells in an amount of 5 µL, and a buffer containing anti-cAMP antibody was added thereto in an amount of 5 µL, and the cells were cultured at room temperature for 15 minutes. Then, a detection mix containing cell lysis buffer was added to the cells in an amount of 10 µL for cell lysis, and reacted at room temperature for 90 minutes. The cell lysate, upon completion of the reaction, was applied to the LANCE cAMP kit (PerkinElmer, USA) to measure the amount of accumulated cAMP, and EC₅₀ values were calculated therefrom and compared with each other. The EC₅₀ values and the relative titers relative to human glucagon and human GLP-1 are shown in Table 10 below.

As shown in Table 10, the heterodimer including imidazo-acetyl exendin-4 and a glucagon analog according to the present invention showed 4.6% of GLP-1 activity and 5.1% of glucagon activity, and these values were confirmed to be similar to those of the long-acting imidazo-acetyl exendin-4 monomer and the long-acting glucagon analog monomer, respectively.

From the above results, it was confirmed that in a case of a conjugate, in which two or more kinds of physiologically active peptide materials are linked to an immunoglobulin Fc by a non-peptide polymer, respectively, the conjugate had a physiological activity similar to that of a conjugate, in which a single kind of a physiologically active peptide material is linked to an immunoglobulin Fc by a non-peptide polymer.

**[Table 10] Comparison of in-vitro activity of imidazo-acetyl exendin-4 and glucagon analog**

| **Material Name** | **cAMP assay (GLP-1)** | | **cAMP assay (Glucagon)** | |
|---|---|---|---|---|
| | **EC₅₀ (nM)** | **Relative activity (% vs. GLP-1)** | **EC₅₀ (nM)** | **Relative activity (% vs. GCG)** |
| **Glucagon-like Peptide-1 (GLP-1)** | 0.025 | **100** | ND | ND |
| **Glucagon (GCG)** | 2.313 | 1.1 | 0.010 | **100** |
| **Monomer Conjugate of CA-Exendin-4** | 0.381 | **6.5** | ND | ND |
| **Monomer Conjugate of Glucagon Analog** | 14.63 | 0.2 | 0.138 | **7.1** |
| **Heterodimer Conjugate of *CA*-Exendin-4/Glucagon Analog** | 0.544 | **4.6** | 0.191 | **5.1** |
| **Immunoglobulin Fc** | ND | ND | ND | ND |

| | | | | |
|---|---|---|---|---|
| ND: Not determined, because the dose-response curve was not saturated up to highest evaluated concentration. | | | | |

From the foregoing, a skilled person in the art to which the present invention pertains will be able to understand that the present invention may be embodied in other specific forms without modifying the technical concepts or essential characteristics of the present invention. In this regard, the specific embodiments disclosed herein are only for illustrative purposes and should not be construed as limiting the scope of the present invention. On the contrary, the present invention is intended to cover not only the specific embodiments but also various alternatives, modifications, equivalents, and other embodiments that may be included within the spirit and scope of the present invention as defined by the appended claims.

## Claims

1. A protein conjugate comprising two or more physiologically active polypeptides,
wherein the protein conjugate comprises:
(i) a unit of an immunoglobulin Fc region or a part of the Fc region having an FcRn-binding affinity;
(ii) a non-peptide polymer covalently linked to the Fc region or the part thereof; and
(iii) a physiologically active polypeptide covalently linked to the non-peptide polymer;
wherein the Fc region or the part thereof is a dimer of two polypeptide chains, and one or more of the non-peptide polymers are linked to each of the two polypeptide chains.

2. The protein conjugate of claim 1, wherein the immunoglobulin Fc region is aglycosylated.

3. The protein conjugate of claim 1, wherein the immunoglobulin Fc region is composed of one to four domains selected from the group consisting of a CH1 domain, a CH2 domain, a CH3 domain, and a CH4 domain.

4. The protein conjugate of claim 1, wherein the immunoglobulin Fc region further comprises a hinge region.

5. The protein conjugate of claim 1, wherein the immunoglobulin Fc region is selected from the group consisting of Fc regions of IgG, IgA, IgD, IgE, IgM, a combination thereof, and a hybrid thereof.

6. The protein conjugate of claim 1, wherein the immunoglobulin Fc region is selected from the group consisting of Fc regions of IgG1, IgG2, IgG3, IgG4, a combination thereof, and a hybrid thereof.

7. The protein conjugate of claim 1, wherein the immunoglobulin Fc region is an IgG4 Fc region.

8. The protein conjugate of claim 7, wherein the immunoglobulin Fc region is a human aglycosylated IgG4 Fc region.

9. The protein conjugate of claim 1, wherein the covalent linkage is formed by reacting a reactive group at one end of a non-peptide polymer selected from the group consisting of an aldehyde group, a maleimide group, and a succinimide derivative with an amino group of the Fc region or the part thereof or an amino group of the physiologically active polypeptide.

10. The protein conjugate of claim 9, wherein the succinimide derivative is succinimidyl propionate, succinimidyl carboxymethyl, hydroxy succinimidyl, or succinimidyl carbonate.

11. The protein conjugate of claim 9, wherein the reactive group at both ends of the non-peptide polymer is an aldehyde group.

12. The protein conjugate of claim 1, wherein one end of the non-peptide polymer is linked to any one selected from a *N*-terminus, a side chain of a lysine residue, and a side chain of an arginine residue of the immunoglobulin Fc, and the other end of the non-peptide polymer is linked to any one selected from a *N*-terminus, a side chain of a lysine residue, a side chain of an arginine residue, a side chain of a histidine residue, and a side chain of a cysteine residue of the physiologically active polypeptide.

13. The protein conjugate of claim 1, wherein the non-peptide polymer is at least one kind selected from the group consisting of polyethylene glycol, polypropylene glycol, an ethylene glycol-polypropylene glycol copolymer, polyoxyethylated polyol, polyvinyl alcohol, a polysaccharide, dextran, polyvinyl ethyl ether, a biodegradable polymer, lipid polymer, fatty acid, chitin, hyaluronic acid, and a combination thereof.

14. The protein conjugate of claim 13, wherein the non-peptide polymer is polyethylene glycol.

15. The protein conjugate of claim 1, wherein the physiologically active polypeptide is selected from the group consisting of hormones, cytokines, enzymes, antibodies, growth factors, transcription factors, blood factors, vaccines, structural proteins, ligand proteins, and receptors.

16. The protein conjugate of claim 15, wherein the physiologically active polypeptide is selected from the group consisting of insulin, glucagon, glucagon-like peptides, oxyntomodulin, exendin-3, exendin-4, fibroblast growth factor 21 (FGF21), human growth hormone (hGH), growth hormone-releasing hormone, growth hormone-releasing peptides, interferons, interferon receptors, colony-stimulating factors, G protein-coupled receptors, interleukins, interleukin receptors, enzymes, interleukin-binding proteins, cytokine-binding proteins, macrophage-activating factors, macrophage peptides, B cell factor, T cell factor, protein A, allergy inhibitors, cell necrosis glycoprotein, immunotoxin, lymphotoxin, tumor necrosis factors, tumor inhibitory factors, metastasis-inducing factors, alpha-1-antitrypsin, albumin, α-lactalbumin, apolipoprotein-E, erythropoietin, highly glycosylated erythropoietin, angiopoietins, hemoglobin, thrombin, thrombin receptor-activating peptides, thrombomodulin, blood factors VII, VIIa, VIII, IX, and XIII, plasminogen-activating factors, fibrin-binding peptides, urokinase, streptokinase, hirudin, protein C, C-reactive protein, renin inhibitors, collagenase inhibitors, superoxide dismutase, leptin, platelet-derived growth factors, epidermal growth factors, epithelial cell growth factors, angiostatin, osteogenic growth factors, osteogenesis-promoting proteins, calcitonin, atriopeptin, cartilage-inducing factors, elcatonin, connective tissue-activating factors, tissue factor pathway inhibitors, follicle-stimulating hormone, luteinizing hormone, luteinizing hormone-releasing hormone, nerve growth factors, parathyroid hormone, relaxin, secretin, somatomedin, insulin-like growth factor, adrenocortical hormone, glucagon, cholecystokinin, pancreatic polypeptides, gastrin-releasing peptides, corticotropin-releasing factor, thyroid-stimulating hormone, autotaxin, lactoferrin, myostatin, receptors, receptor antagonists, cell surface antigens, virus-derived vaccine antigens, monoclonal antibody, polyclonal antibody, antibody fragments, erythropoietic growth factors, leukopoietin, amylin, somatostatin, peptide YY (PYY), neuropeptide Y (NPY), angiotensin, bradykinin, calcitonin, corticotropin, eledoisin, gastrin, leptin, oxytocin, vasopressin, luteinizing hormone, luteotropin, follicle-stimulating hormone, parathyroid hormone, secretin, sermorelin, human growth hormone (hGH), growth hormone-releasing peptides, granulocyte colony-stimulating factors (GCSF), interferons (IFN), interleukins, prolactin-releasing peptides, orexin, thyroid-releasing peptides, cholecystokinin, gastrin inhibitory peptides, calmodulin, gastric-releasing peptides, motilin, vasoactive intestinal peptides, atrial natriuretic peptides (ANPs), barin natriuretic peptides (BNPs), C-type natriuretic peptides (CNPs), neurokinin A, neuromedin, renin, endothelin, sarafotoxin peptide, carsomorphin peptide, dermorphin, dynorphin, endorphin, enkepalin, T cell factor, tumor necrosis factors, tumor necrosis factor receptors, urokinase receptors, tumor inhibitory factors, collagenase inhibitors, thymopoietin, thymulin, thymopentin, thymosin, thymic humoral factor, adrenomedullin, allatostatin, amyloid beta-protein fragments, antibacterial peptides, antioxidant peptides, bombesin, osteocalcin, CART peptides, E-selectin, ICAM-1, VCAM-1, leucokine, kringle-5, laminin, inhibin, galanin, fibronectin, pancreastatin, fuzeon, and analogs thereof.

17. The protein conjugate of claim 16, wherein the physiologically active polypeptide is selected from the group consisting of insulin, glucagon, glucagon-like peptides, oxyntomodulin, exendin-3, exendin-4, fibroblast growth factor 21 (FGF21), human growth hormone, interferon-alpha, granulocyte colony-stimulating factors (GCSF), erythropoietin, Fab' antibody fragments, and an analog thereof.

18. The protein conjugate of claim 1, wherein the physiologically active polypeptides linked to each of the two polypeptide chains of the Fc region or a part thereof differ from each other.

19. The protein conjugate of claim 1, wherein each of two units of the physiologically active polypeptides is linked to a single unit of the immunoglobulin Fc region or a part thereof by a non-peptide polymer, and the kinds of the two units are different each other.

20. The protein conjugate of claim 1, wherein the physiologically active polypeptides of different kinds are linked to a single unit of the immunoglobulin Fc region or a part thereof by a non-peptide polymer, respectively, and the physiologically active polypeptides of different kinds are present in a different ratio.

21. The protein conjugate of claim 1, wherein the physiologically active polypeptide, which is covalently linked to the immunoglobulin Fc region or a part thereof by a non-peptide polymer, is further linked with another physiologically active polypeptide, in a site other than the site to which the non-peptide polymer is linked.

22. The protein conjugate of claim 1, wherein each of three or more units of physiologically active polypeptides is linked to a single unit of the immunoglobulin Fc region or a part thereof by a non-peptide polymer, and the kinds of the three or more units are different from each other.

23. A homomultimer conjugate of physiologically active polypeptides, wherein each of three or more units of physiologically active polypeptides is covalently linked to a single unit of the immunoglobulin Fc region or the part thereof having the FcRn-binding affinity, by a non-peptide polymer and wherein the three or more units exhibit the same physiological activity.

24. A method for preparing a protein conjugate of the physiologically active polypeptide according to any of claims 1 to 22, comprising:
providing (a) a non-peptide polymer, which is linked at one end to a physiologically active polypeptide and comprises a remaining reactive group at the other end, and (b) a physiologically active polypeptide conjugate, in which one or more physiologically active polypeptides are respectively linked to a unit of the immunoglobulin Fc region or a part thereof having the FcRn-binding affinity, wherein the unit of the immunoglobulin Fc region or a part thereof having the FcRn-binding affinity comprises a remaining reactive group; and
covalently linking the remaining reactive group of the non-peptide polymer, which is linked to the physiologically active polypeptide of (a), and the remaining reactive group of the immunoglobulin Fc region or a part thereof of the physiologically active polypeptide conjugate of (b).

25. The method of claim 24, wherein the non-peptide polymer of (a) comprises a remaining reactive group selected from the group consisting of an aldehyde group, a maleimide group, and a succinimide derivative.

26. The method of claim 24, wherein the remaining reactive group in the immunoglobulin Fc region or a part thereof of (b) is positioned at one position selected from a *N*-terminus, a side chain of a lysine residue, and a side chain of an arginine residue of the immunoglobulin Fc region or a part thereof.

27. The method of claim 24, wherein the physiologically active polypeptide of (a) and the physiologically active polypeptide of (b) are of the same or different kind.

28. The method of claim 24, wherein the physiologically active polypeptide of (a) and the physiologically active polypeptide of (b) are in a single unit.
